# EUROPEAN PATENT APPLICATION

(11) **EP 4 242 220 A1**
(43) Date of publication of application: **13.09.2023**
(21) Application number: 21889053.1
(22) Date of filing: 22.10.2021
(51) Int. Cl.: C07K 1/22, A61K 45/00, A61K 47/62, A61P 43/00, C07C 49/16, C07K 1/10, C07K 2/00, C07K 7/06, C07K 7/08, C07K 14/00, C07K 16/00, C07K 17/00, C12P 21/00

(54) **PEPTIDE CROSSLINKING AGENT AND CROSSLINKED PEPTIDE WHICH IS CROSSLINKED USING SAID CROSSLINKING AGENT**

(30) Priority: 09.11.2020 JP 2020186833; 14.05.2021 JP 2021082739
(71) Applicant: Kagoshima University, Kagoshima-shi, Kagoshima 890-8580 (JP)
(72) Inventor: ITO Yuji, Kagoshima-shi, Kagoshima 890-8580 (JP); NAKAYAMA Hiroshi, Kagoshima-shi, Kagoshima 890-8580 (JP); RAFIQUE Md Abdur, Kagoshima-shi, Kagoshima 890-8580 (JP)
(74) Representative: J A Kemp LLP
(86) International application number: PCT/JP2021/039054
(87) International publication number: WO 2022/097500

(57) **Abstract**

A crosslinking agent for a protein or a peptide is represented by the following formula (I). In the formula, A is a hydrogen atom, a C1 to 6 alkyl group optionally substituted with a phenyl group or a halogen atom, or a phenyl group.

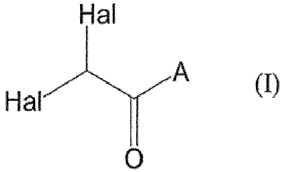

## Description

### Technical Field

The present disclosure relates to a method for producing a crosslinked peptide, and the like.

### Background Art

Protein pharmaceuticals and peptide pharmaceuticals are one of biopharmaceutical products currently drawing the most attention. In particular, antibody pharmaceuticals, mainly IgG antibodies, and peptide pharmaceuticals have been recently utilized in the pharmaceutical field, and have been increasingly important for industrial and pharmaceutical applications.

The inventor has heretofore reported the ability of purification of IgG by a column where a peptide ligand containing an IgG peptide composed of 17 residues and cyclized by a disulfide bond and containing a specified sequence is immobilized, in order to construct an alternative system to a protein A column used for purification of an antibody pharmaceutical product (Patent Literature 1). However, such an IgG peptide column has the problem of being low in alkaline resistance of a disulfide bond present in the peptide and thus unable to be repeatedly utilized. The inventor has repeatedly studied solutions for increasing alkali resistance in order to solve the problem of chemical weakness of a disulfide bond in a peptide or protein, against alkali washing, and as a result, has found an IgG-binding peptide dramatically increased in resistance against alkali washing due to crosslinking between thiol groups in a cysteine residue in a peptide or protein by use of 1,3-dichloroacetone (Patent Literature 2). However, this crosslinking, although imparts strong alkali resistance, has the new problem of causing a low affinity (Kd = 4.9 µM) with IgG and the loss of functionality. Then, a crosslinked structure has been re-designed and thus tried to be improved so as to allow for retention of alkali resistance without any considerable deterioration in functionality, unlike crosslinking by dichloroacetone, and as a result, it has been found that one thiol in disulfide is changed to a methyl group to convert an SS bond into a CH₃-S bond, thereby providing a crosslinked structure close to practical one, in which the crosslinked structure, although is still slightly low in affinity, has a Kd value of 340 nM and has alkali resistance (Patent Literature 3). However, such a method involves chlorinating homoserine in a peptide once and then reacting the resultant with a thiol group of cysteine, and thus has the problem of being low in production yield.

### Citation List

### Patent Literature

Patent Literature 1: International Publication No. WO 2013/027796
Patent Literature 2: International Publication No. WO 2018/092867
Patent Literature 3: International Publication No. WO 2020/075670

### Summary of Invention

### Technical Problem

An objective of the present disclosure is to provide a method for producing a crosslinked structure with a high yield that is high in alkali resistance and retained in functionality without any considerable deterioration in affinity, by re-design of a crosslinked structure.

### Solution to Problem

The inventors have further studied improvements of structures of crosslinking agents, and as a result, has succeeded in producing an IgG-binding peptide retaining alkali resistance and a high affinity (Kd = 45 nM) with IgG by using of 1,1-dichloroacetone and its derivative that are usually not used in an aqueous solution due to its high reactivity, as a crosslinking agent. This disclosure has succeeded in providing a crosslinking technique that can be practically used for pharmaceutical products, that imparts a high yield, and that can allow the function of a protein or peptide to be kept with alkali resistance being exhibited.

A crosslinking agent for a protein or a peptide according to a first aspect of the present disclosure is represented by the following formula (I).

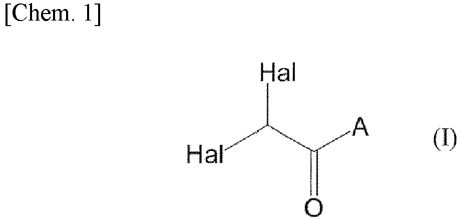

In the formula, A is a hydrogen atom, a C1 to 6 alkyl group optionally substituted with a phenyl group or a halogen atom, or a phenyl group.

A method for producing a crosslinked protein or peptide according to a second aspect of the present disclosure is a method for producing a crosslinked protein or peptide in which at least two thiol groups in a single or separated protein(s) or peptide(s) are bound as represented by the following formula: 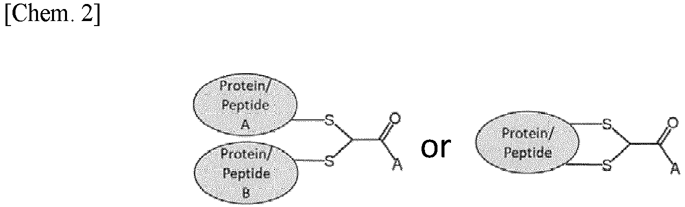
[wherein A is a hydrogen atom, a C1 to 6 alkyl group optionally substituted with a phenyl group or a halogen atom, a phenyl group, or a C1 to 6 alkyl group, Protein/Peptide represents a protein or a peptide, and Protein/Peptide A and Protein/Peptide B are optionally the same or different;]
   comprising
   reacting the protein or the peptide with the crosslinking agent according to the first aspect of the present disclosure, to bind the two thiol groups via the following group: 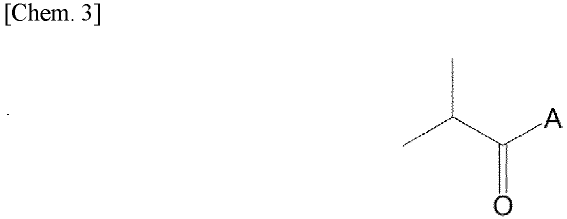
[wherein A is a hydrogen atom, a C1 to 6 alkyl group optionally substituted with a phenyl group or a halogen atom, a phenyl group, or a C1 to 6 alkyl group].

The protein or the peptide may be a protein or a peptide wherein all or a part of the at least two thiol groups form a disulfide bond, and the method may comprise reducing the disulfide bond to thereby generate two thiol groups.

A method for improving alkali resistance of a protein or a peptide according to a third aspect of the present disclosure comprises binding a thiol groups in the protein or the peptide to obtain a crosslinked protein or peptide by the method according to the second aspect of the present disclosure.

A method for producing a protein or a peptide according to a fourth aspect of the present disclosure is a method for producing a protein or a peptide bound to a reactive functional group which is represented by the following formula: 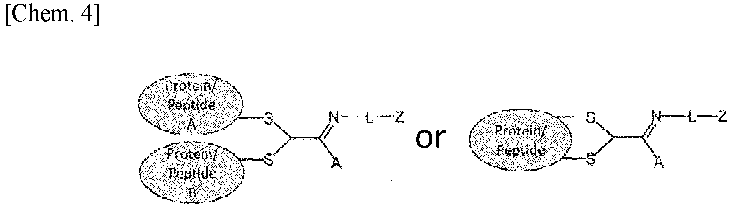 [wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and Z represents the reactive functional group;]
comprising:
obtaining the crosslinked protein or peptide by the method according to the second aspect of the present disclosure; and
reacting the crosslinked protein or peptide obtained, with NH₂-L-Z (L represents a linker and Z represents the reactive functional group), to thereby introduce a reactive functional group into a crosslinked portion of the crosslinked protein or peptide.

A method for producing a protein(s) or a peptide(s) according to a fifth aspect of the present disclosure is a method for producing a protein(s) or a peptide(s) bound to a reactive functional group which is represented by the following formula: 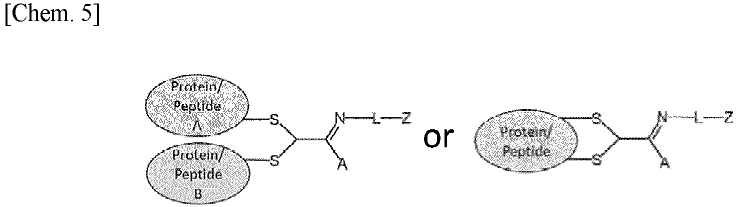
[wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and Z represents the reactive functional group;]
   comprising:
   reacting a crosslinked protein(s) or peptide(s) represented by the following formula: 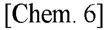 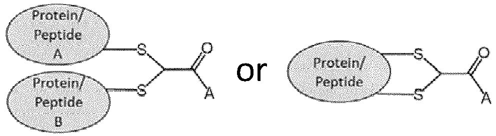
[wherein A is a hydrogen atom, a C1 to 6 alkyl group optionally substituted with a phenyl group or a halogen atom, a phenyl group, or a C1 to 6 alkyl group, Protein/Peptide represents a protein or a peptide, and Protein/Peptide A and Protein/Peptide B are optionally the same or different;]
and NH₂-L-Z (L represents a linker and Z represents the reactive functional group), to thereby introduce the reactive functional group to a crosslinked portion of the crosslinked protein(s) or peptide(s).

A method for producing a complex of a protein(s) or peptide(s) and a drug according to a sixth aspect of the present disclosure is a method for producing a complex of a protein(s) or peptide(s) and a drug represented by the following formula: 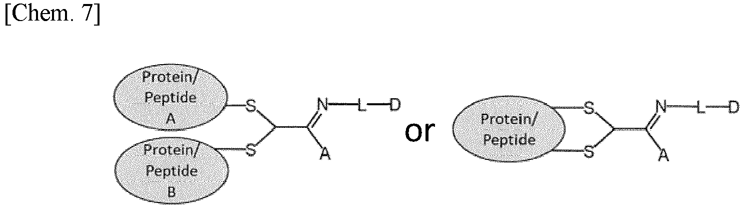 [wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and D represents the drug;]
comprising:
obtaining a protein(s) or a peptide(s) bound to a reactive functional group by the method according to the fourth or fifth aspect of the present disclosure; and
reacting the obtained protein(s) or peptide(s) bound to the reactive functional group and the drug having another functional group reactive with the reactive functional group, to thereby bind the drug to a crosslinked portion of the crosslinked protein(s) or peptide(s).

A method for producing a complex of a protein(s) or a peptide(s) and a drug according to a seventh aspect of the present disclosure is a method for producing a complex of a protein(s) or a peptide(s) and a drug represented by the following formula: 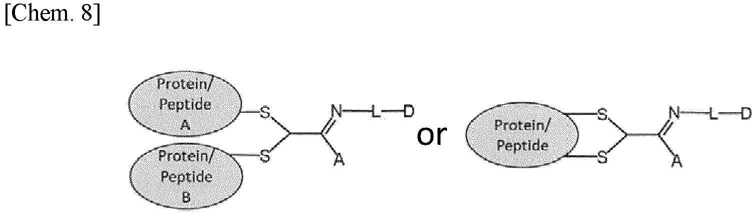
[wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and D represents the drug;]
   comprising:
   reacting a protein(s) or a peptide(s) bound to a reactive functional group which is represented by the following formula: 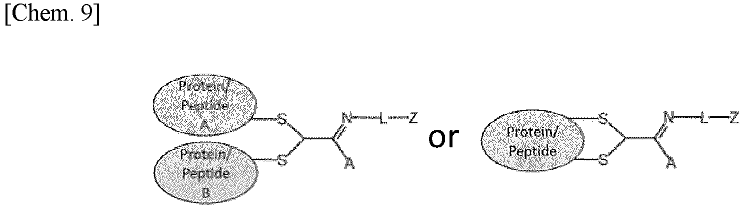
[wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and Z represents the reactive functional group;]
and the drug having another functional group reactive with the reactive functional group, to thereby bind the drug to a crosslinked portion of the crosslinked protein(s) or peptide(s).

In the methods according to the fourth to seventh aspects of the present disclosure, the linker may contain a moiety cleavable by a proteolytic enzyme.

In the methods according to the second to seventh aspects of the present disclosure, the protein or the peptide may be a peptide of 5 to 50 amino acids.

In the methods according to the second to seventh aspects of the present disclosure, the thiol groups for crosslinking may be present in a single protein or peptide.

In the methods according to the second to seventh aspects of the present disclosure, the protein or peptide may be an Fc-binding peptide.

In the methods according to the second to seventh aspects of the present disclosure, the thiol groups for crosslinking may be present in a separated proteins or peptides.

A crosslinked protein(s) or peptide(s) according to an eighth aspect of the present disclosure is represented by the following formula. 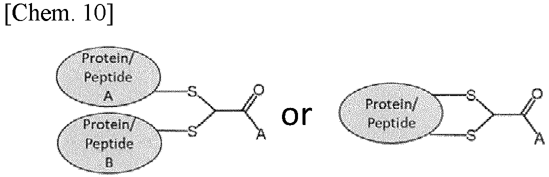 [wherein A is a hydrogen atom, a C1 to 6 alkyl group optionally substituted with a phenyl group or a halogen atom, a phenyl group, or a C1 to 6 alkyl group, Protein/Peptide represents a protein or a peptide, and Protein/Peptide A and Protein/Peptide B are optionally the same or different].

A protein(s) or a peptide(s) bound to a reactive functional group according to a ninth aspect of the present disclosure is represented by the following formula: 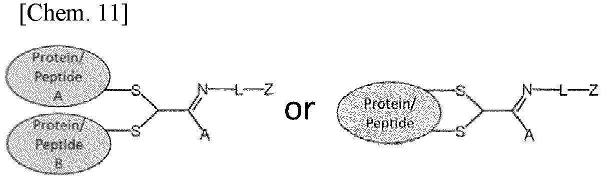 [wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and Z represents the reactive functional group.

A complex of a protein(s) or a peptide(s) and a drug according to a tenth aspect of the present disclosure is represented by the following formula: 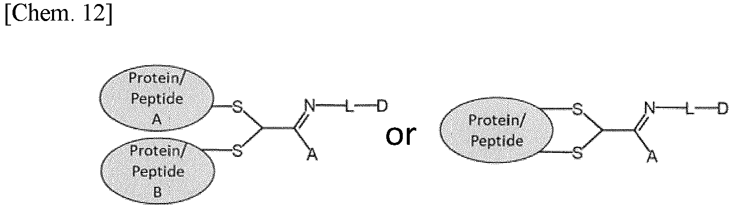 [wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and D represents the drug].

In the protein(s) or peptide(s) according to the eighth and ninth aspects of the present disclosure, and the complex according to the tenth aspect of the present disclosure, the linker may contain a moiety cleavable by a proteolytic enzyme.

In the proteins or peptides according to the eighth and ninth aspects of the present disclosure, and the complex according to the tenth aspect of the present disclosure, the protein or the peptide may be a peptide of 5 to 50 amino acids.

In the protein or the peptides according to the eighth and ninth aspects of the present disclosure, and the complex according to the tenth aspect of the present disclosure, the thiol groups for crosslinking may be present in a single protein or peptide.

In the proteins or the peptides according to the eighth and ninth aspects of the present disclosure, and the complex according to the tenth aspect of the present disclosure, the protein or the peptide may be an Fc-binding peptide.

An amino group in a lysine residue, a cysteine residue, an aspartic acid residue, a glutamic acid residue, 2-aminosuberic acid, diaminopropionic acid, an arginine residue or an amino acid at the 1-position in the Fc-binding peptide, may be modified with DSG (disuccinimidyl glutarate), DSS (disuccinimidyl suberate), DMA (dimethyl adipimidate dihydrochloride), DMP (dimethyl pimelimidate dihydrochloride), DMS (dimethyl suberimidate dihydrochloride), DTBP (dimethyl 3,3'-dithiobispropionimidate dihydrochloride), or DSP (dithiobis(succinimidyl propionic acid)).

In the proteins or the peptides according to the eighth and ninth aspects of the present disclosure, and the complex according to the tenth aspect of the present disclosure, the thiol groups for crosslinking may be present in a separated proteins or peptides.

A method for producing an IgG-Fc region-containing molecule bound to a crosslinked Fc-binding peptide(s) according to an eleventh aspect of the present disclosure comprises contacting an IgG-Fc region-containing molecule and the protein or the peptide according to the eighth or ninth aspect of the present disclosure or the complex according to the tenth aspect of the present disclosure.

A method for producing an IgG-Fc region-containing molecule bound to a crosslinked Fc-binding peptide(s) according to a twelfth aspect of the present disclosure comprises
reacting an Fc-binding peptide bound to an IgG-Fc region-containing molecule, and the crosslinking agent according to the first aspect of the present disclosure, to bind two thiol groups in the Fc-binding peptide via the following group: 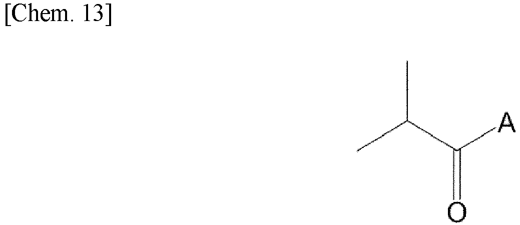
[wherein A is a hydrogen atom, a C1 to 6 alkyl group optionally substituted with a phenyl group or a halogen atom, a phenyl group, or a C1 to 6 alkyl group].

A method for producing an IgG-Fc region-containing molecule bound to an Fc-binding peptide having a reactive functional group Z,
wherein the Fc-binding peptide is represented by the following formula, according to a thirteenth aspect of the present disclosure: 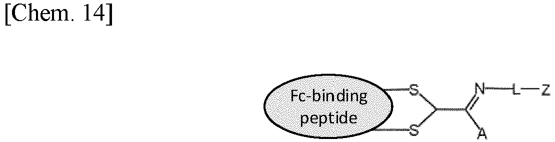
[wherein A is a hydrogen atom, a C1 to 6 alkyl group optionally substituted with a phenyl group or a halogen atom, a phenyl group, or a C1 to 6 alkyl group, L represents a linker and Z represents the reactive functional group;]
   comprises
   reacting an IgG-Fc region-containing molecule bound to a crosslinked Fc-binding peptide represented by the following formula: 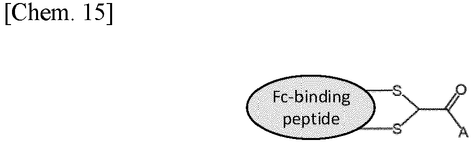
[wherein A is a hydrogen atom, a C1 to 6 alkyl group optionally substituted with a phenyl group or a halogen atom, a phenyl group, or a C1 to 6 alkyl group;]
and NH₂-L-Z (L represents a linker and Z represents the reactive functional group), to thereby introduce the reactive functional group into a crosslinked portion in the crosslinked Fc-binding peptide.

A method for producing an IgG-Fc region-containing molecule bound to an Fc-binding peptide having a drug D,
wherein the Fc-binding peptide is represented by the following formula, according to a fourteenth aspect of the present disclosure, 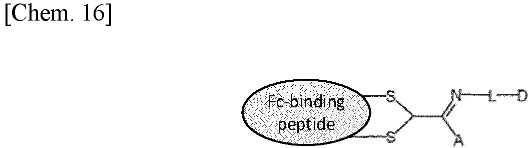 comprises
   reacting an IgG-Fc region-containing molecule bound to an Fc-binding peptide(s) having a reactive functional group Z,
wherein the Fc-binding peptide is represented by the following formula: 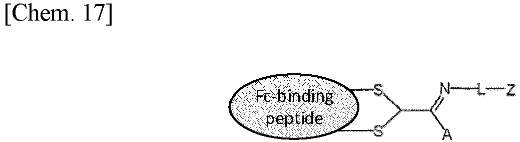
[wherein A is a hydrogen atom, a C1 to 6 alkyl group optionally substituted with a phenyl group or a halogen atom, a phenyl group, or a C1 to 6 alkyl group, L represents a linker and Z represents the reactive functional group;]
and the drug having another functional group reactive with the reactive functional group Z, to thereby bind the drug to a crosslinked portion of a crosslinked protein or peptide.

The methods according to the eleventh to fourteenth aspects of the present disclosure may each further comprise covalently binding the Fc-binding peptide and the IgG-Fc region-containing molecule.

An IgG-Fc region-containing molecule according to a fifteenth aspect of the present disclosure is to which the proteins or the peptides according to the eighth and ninth aspects of the present disclosure or the complex according to the tenth aspect of the present disclosure is bound.

In the molecule according to the fifteenth aspect of the present disclosure, the Fc-binding peptide and the IgG-Fc region-containing molecule may be covalently bound.

A carrier according to a sixteenth aspect of the present disclosure is to which the peptide according to the eighth or ninth aspect of the present disclosure is bound.

A method for purifying an IgG-Fc region-containing molecule according to a seventeenth aspect of the present disclosure comprises
contacting a liquid containing an IgG-Fc region-containing molecule, with the carrier according to the sixteenth aspect of the present disclosure,
removing a component not binding to the carrier, by washing, and
eluting a component bound to the carrier, to recover the IgG-Fc region-containing molecule.

A method for producing a crosslinked protein or peptide according to an eighteenth aspect of the present disclosure is a method for producing a crosslinked protein or peptide in which at least two thiol groups are bound as,
wherein the crosslinked protein or peptide is represented by the following formula: 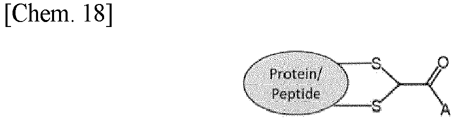
[wherein A is a hydrogen atom, a C1 to 6 alkyl group optionally substituted with a phenyl group or a halogen atom, a phenyl group, or a C1 to 6 alkyl group, and Protein/Peptide represents a protein or a peptide;]
   comprising:
   synthesizing the protein or peptide by an Fmoc method; and
   using a compound represented by the following formula, instead of at least one cysteine residue, in the synthesis.

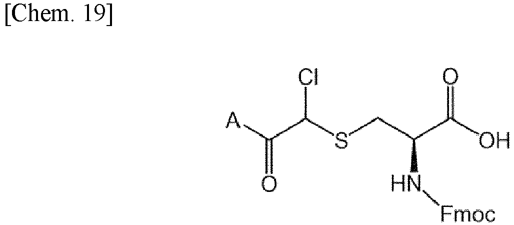

A method for producing a protein or a peptide according to a nineteenth aspect of the present disclosure is a method for producing a protein or a peptide bound to a reactive functional group represented by the following formula: 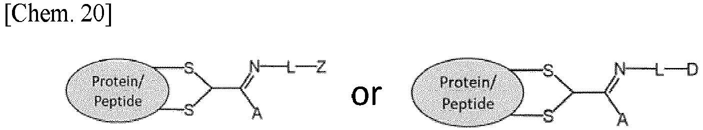 [wherein A is a hydrogen atom, a C1 to 6 alkyl group optionally substituted with a phenyl group or a halogen atom, a phenyl group, or a C1 to 6 alkyl group, Protein/Peptide represents a protein or peptide, L represents a linker, Z represents a reactive functional group and D represents a drug;]
comprising:
synthesizing the protein or peptide by an Fmoc method; and
using a compound represented by the following formula, instead of at least one cysteine residue, in the synthesis.

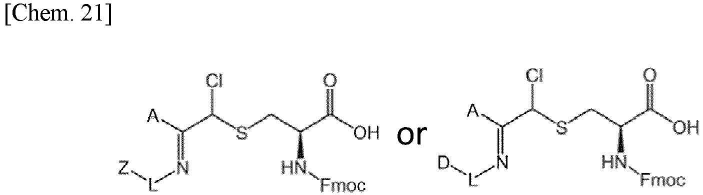

### Advantageous Effects of Invention

An SH-SH bond in an amino acid obtained by the method of the present disclosure can form a crosslinked structure at a high yield. The bond is excellent in alkali resistance, and thus is not cleaved even if exposed under alkaline conditions. Accordingly, a protein and a peptide having strong alkali resistance can be provided, and these can be used as a protein and a peptide which can be used or washed under alkaline conditions. Furthermore, such a protein and a peptide keep the structures originally possessed therein by the crosslinked structures and are not impaired in functions, and thus can be used for stabilization of various peptides and proteins.

### Brief Description of Drawings

FIG. 1 schematically illustrates an intramolecular SS bond; and A illustrates an SS bond in a natural protein or peptide, B illustrates a crosslinked bond in a conventional technique, and C illustrates a crosslinked bond in the present disclosure;
FIG. 2 is a graph representing the results of evaluation of DBC with a 1,1-dichloroacetone-crosslinked peptide derivative-immobilized column; the vertical axis represents the absorbance at 280 nm, the horizontal axis represents the elution time, the dashed line represents the breakthrough point at 10%, the solid line represents the results of alkali washing once to 5 times (dark line represents a larger number of washing times), and the dotted line represents the results of alkali washing 10 times to 30 times (dark line represents a larger number of washing times);
FIG. 3 is a graph representing the change in DBC of a peptide-immobilized column by alkali washing; and the vertical axis represents the variation (%) and the horizontal axis represents the number of washing times;
FIG. 4 schematically illustrates the crosslinked peptide of the present disclosure;
FIG. 5 includes diagrams representing the analysis results before crosslinking reaction and after crosslinking reaction of oxytocin in Example 11, with a liquid chromatography-mass analyzer (LC-MS); and A and B are respectively diagrams representing elution chromatograms of an oxytocin SS oxidized form before crosslinking reaction and a reaction product after crosslinking reaction;
FIG. 6 includes diagrams representing the analysis results before crosslinking reaction and after crosslinking reaction of vasopressin in Example 12, with LC-MS; and A and B are respectively diagrams representing elution chromatograms of a vasopressin SS oxidized form before crosslinking reaction and a reaction product after crosslinking reaction;
FIG. 7 includes diagrams representing the analysis results of an oxytocin SS oxidized form exposed to α-chymotrypsin in Example 13, with reverse-phase high-performance liquid chromatography (HPLC);
FIG. 8 includes diagrams representing the analysis results of an oxytocin SH reduced form exposed to α-chymotrypsin in Example 13, with reverse-phase HPLC;
FIG. 9 includes diagrams representing the analysis results of a reaction product of a 2,2-dichloroacetophenone crosslinked form exposed to α-chymotrypsin in Example 13, with reverse-phase HPLC; and
FIG. 10 includes diagrams representing the amount of change with respect to each kind of molecules after addition of α-chymotrypsin in Example 13.

### Description of Embodiments

### (Crosslinking agent)

A crosslinking agent for crosslinking a protein or peptide in one aspect is represented by the following formula (I).

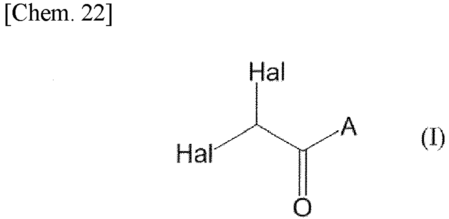

In the formula, Hal represents a halogen atom, two halogen atoms are optionally the same or different, and A is a hydrogen atom, a C1 to 6 alkyl group optionally substituted with a phenyl group or a halogen atom, or a phenyl group.

Herein, the "alkyl group" means a straight, branched, or cyclic saturated hydrocarbon group. Herein, C represents the number of carbon atoms, and "C1 to 6 alkyl group" represents an alkyl group having 1 to 6 carbon atoms. Examples of the alkyl group include a methyl group, an ethyl group, a n-propyl group, an i-propyl group, a n-butyl group, a sec-butyl group, a t-butyl group, an isobutyl group, a pentyl group, an isopentyl group, a 2,3-dimethylpropyl group, a hexyl group, and a cyclohexyl group.

The number of substituents in the C1 to 6 alkyl group in the group A can be 1 to 10, 1 to 8, 1 to 6, 1 to 5, 1 to 4, 1 to 3, 3, 2, and/or 1, and, for example, trifluoromethane can be exemplified.

The "halogen atom" means a fluorine atom, a chlorine atom, a bromine atom, and an iodine atom, preferably a fluorine atom, a chlorine atom, and a bromine atom, more preferably a fluorine atom or a chlorine atom.

Preferably, two Hals are the same halogen atoms, and both may be, for example, fluorine atoms, chlorine atoms, or bromine atoms.

Examples of the crosslinking agent can include 1,1-dichloroacetone, 1,1-dichloropinacolin, and 2,2-dichloroacetophenone.

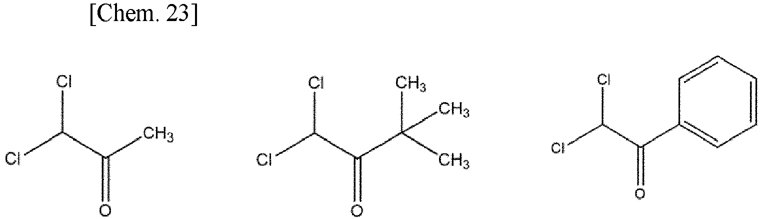

### (Method for synthesizing crosslinking agent)

When A is an alkyl chain, dichlorination can be made by synthesis according to the Document of Gallucci et al. (Gallucci, R. R. and Going, R., Journal of Organic Chemistry, 1981, vol. 46, #12, p. 2532-2538). On the other hand, when A is phenyl, dichlorination can be made by synthesis according to the Document of Zhang et al. (Shao-Lin Zhang, Zheng Yang, Xiaohui Hu, Kin Yip Tam, Bioorganic & Medicinal Chemistry Letters, vol. 28, Issue 21, 15 November 2018, p. 3441-3445).

### (Method for producing crosslinked protein or peptide)

In one aspect, the method for producing a crosslinked protein or peptide is a method for producing a crosslinked protein or peptide where at least two thiol groups in a single or separated protein or peptide are bound, the crosslinked protein or peptide being represented by the following formula: 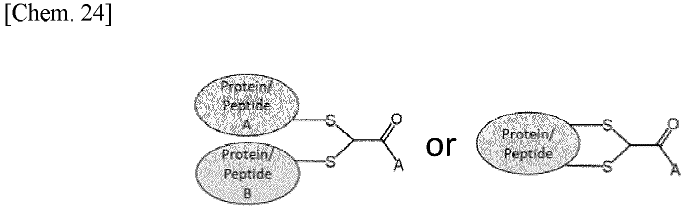
wherein A is a hydrogen atom, a C1 to 6 alkyl group optionally substituted with a phenyl group or a halogen atom, a phenyl group, or a C1 to 6 alkyl group, Protein/Peptide represents a protein or peptide, and Protein/Peptide A and Protein/Peptide B are optionally the same or different;
the method including:
   reacting a protein or peptide with the crosslinking agent according to the present embodiment, to bind the two thiol groups via the following group: 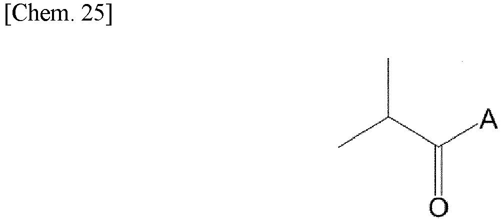
wherein A is a hydrogen atom, a C1 to 6 alkyl group optionally substituted with a phenyl group or a halogen atom, a phenyl group, or a C1 to 6 alkyl group.

The method for producing a crosslinked protein can be represented by, for example, the following reaction scheme.

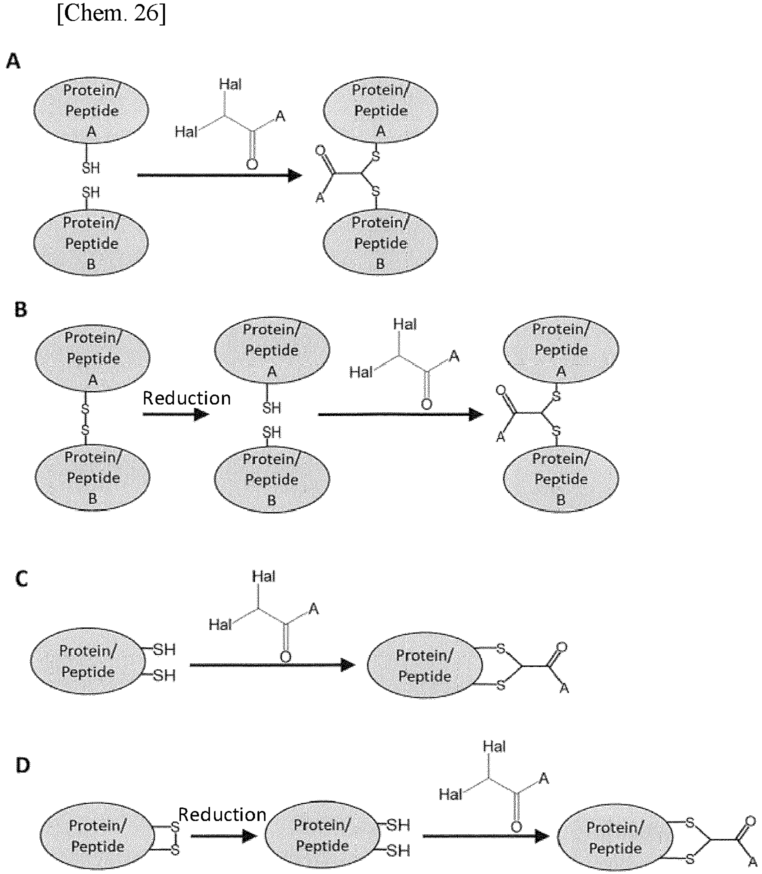

The crosslinking reaction between the crosslinking agent and the thiol groups can be performed by adding the crosslinking agent dissolved in acetonitrile, to a protein or peptide solution where an SH group is generated by cleavage of a disulfide bond due to addition of TCEP-HCl, if necessary, dissolved in buffer such as PBS and reduction under stirring at room temperature for 30 minutes to one day, and then stirring the mixture at room temperature for 30 minutes to one day. The reaction solution can be, if necessary, purified with HPLC or the like. The protein or peptide serving as a raw material may be, if necessary, protected with Fmoc, and in this case, a base such as piperidine is added in order to deprotect an Fmoc protection group in the protein or peptide, after the reaction. The reaction solution can be, if necessary, further purified with HPLC (C18 reverse-phase column). Whether or not a crosslinked protein or peptide is generated can be confirmed by confirmation of the molecular weight with, for example, LC-MS analysis.

The crosslinking agent according to the present embodiment may impart crosslinking of SH groups present in two proteins or peptides separated, and in this case, such two proteins or peptides are reacted with the crosslinking agent and thus bound (A). Such two molecules are optionally the same or different kinds of molecules. Alternatively, the present crosslinking method may involve crosslinking two SH groups present in one molecule, and in this case, one protein or peptide is reacted with the crosslinking agent to thereby perform crosslinking (C).

The crosslinking agent herein imparts crosslinking of two thiol groups. The "thiol group" or the "SH group" is usually derived from a cysteine residue constituting a protein or peptide, and may be a group artificially introduced, for example, by use of an artificial amino acid. The two thiol groups, which are in the natural state or the state before crosslinking, may or may not form a disulfide bond.

When the two thiol groups, which are in the natural state or the state before crosslinking, form a disulfide bond, a step of reducing the disulfide bond to generate thiol groups before the crosslinking method may be included (B and D). For example, the disulfide bond can be reduced by using, for example, a commercially available reducing agent for protein disulfide, such as tris(2-carboethoxy)phosphine (TCEP), a hydrochloride thereof, dithioethanol (DTT), 2-mercaptoethanol, and cysteine hydrochloride (Cys-HCl). The two thiol groups may be thiol groups present in a cysteine residue contained in a single protein or peptide, or may be thiol groups of separated proteins or peptides (including proteins, peptides, and a combination of a protein and a peptide). When thiol groups forming a disulfide bond in a single protein or peptide are crosslinked, the structure after the crosslinking desirably provides the same steric structure as in the disulfide bond.

The protein or peptide to be crosslinked each has at least two SH groups in the case of intramolecular crosslinking, or at least one SH group in the case of intermolecular crosslinking. The protein or peptide may have, for example, one or more (for example, 1 to 10, 2 to 8, 4 to 6, 1, 2, 3, 4, 5, or 6) disulfide bonds in a molecule or between molecules. For example, the protein may be a protein of 500 Da or more, 1000 Da or more, 2000 Da or more, and/or 500,000 Da or less, 200,000 Da or less, or 100,000 Da or less. For example, the protein may be an enzyme, a glycoprotein (erythropoietin or the like), cytokine, toxin, an adjuvant, a structure protein, an antibody, an Fc fusion protein, an antibody fragment (F(ab')₂, Fab', Fab, Fabs, single-stranded Fv(scFv), (tandem) bispecific single-stranded Fv(sc(Fv)₂), a single-stranded triplebody, a nanobody, a divalent VHH, a pentavalent VHH, a minibody, a (double-stranded) diabody, a tandem diabody, a bispecific tribody, a bispecific bibody, a dual affinity retargeting molecule (DART), a triabody (or tribody), a tetrabody (or [sc(Fv)₂]₂), or (scFv-SA)₄) disulfide bond Fv, compact IgG, a heavy chain antibody, or a polymer thereof). The peptide may be, for example, a peptide of 5 to 5000 amino acids, 5 to 1000 amino acids, 5 to 500 amino acids, 5 to 100 amino acids, 5 to 50 amino acids, or 10 to 40 amino acids. The peptide may be either cyclic or linear. Examples of the peptide include vaccine, a micro antibody, and an antibacterial peptide. Herein, the protein and the peptide each containing an antibody Fc region are referred to as "antibody or the like".

The crosslinking method may form a single or same crosslinked structure, or a plurality of crosslinked structures. A plurality of proteins or peptides may be crosslinked and thus the same or different three or more proteins and/or peptides may be crosslinked. Such crosslinking may be made by a combination of intramolecular crosslinking and intermolecular crosslinking, and, for example, may be made by two kinds of crosslinking, intramolecular crosslinking in an Fc-binding peptide described below and intermolecular crosslinking between the peptide and the antibody Fc region.

### (Method for producing crosslinked protein or peptide by peptide synthesis)

Alternatively, a crosslinking method according to another aspect is a method for producing a crosslinked protein or peptide where at least two thiol groups in a protein or peptide are bound, the crosslinked protein or peptide being represented by the following formula: 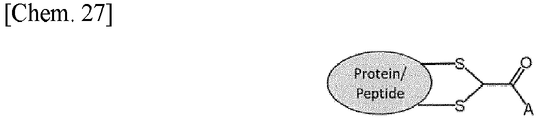 wherein A is a hydrogen atom, a C1 to 6 alkyl group optionally substituted with a phenyl group or a halogen atom, a phenyl group, or a C1 to 6 alkyl group, and Protein/Peptide represents a protein or peptide;
the method including:
synthesizing the protein or peptide by an Fmoc method; and
using a compound represented by the following formula, instead of at least one cysteine residue, in the synthesis. 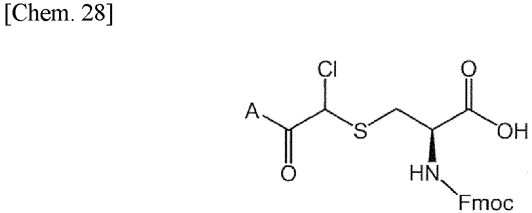
More specifically, the present method involves synthesizing a peptide containing two Cyses to be crosslinked, from the C-terminal, on a carrier such as a peptide synthesis resin according to an Fmoc method. The first Cys is bound by using common Cys (if necessary, an SH group is optionally protected), and subjected to synthesis up to the previous amino acid of the remaining other Cys, according to a common Fmoc method. If Cys contained in a peptide being synthesized is protected, such a protection group is deprotected and then Fmoc-cysteine (example: Fmoc-chloroacetophenoyl cysteine) where a 1,1-dichloroacetone derivative (for example, 1,1-dichloroacetone, 1,1-dichloropinacolin, or 2,2-dichloroacetophenone) is bound on an SH group is added and linked (step d in scheme B). Fmoc is deprotected (step e in scheme B) and an α-amino group at the N-terminal of the peptide generated and an α-carboxyl group at the acetophenoyl cysteine side are coupled (step f in scheme B), and thus the cysteine where the 1,1-dichloroacetone derivative is bound on an SH group is bound to a peptide chain. The remaining amino acid is synthesized by linking according to an Fmoc method (step g in scheme B), and thus a peptide chain crosslinked is completed.

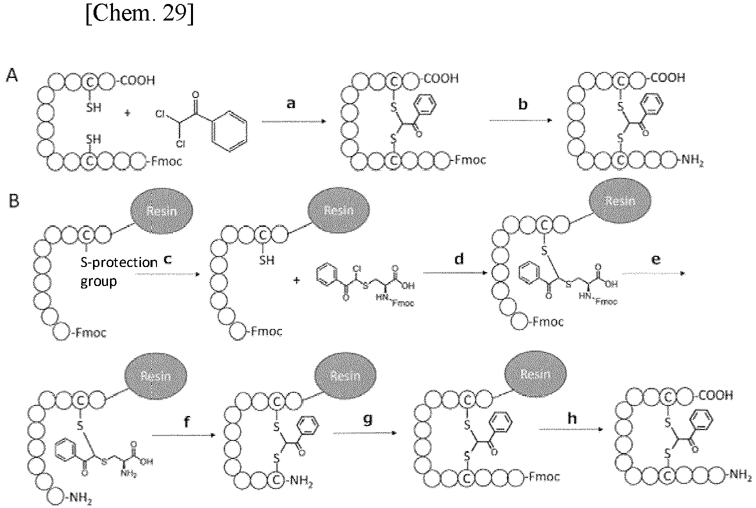

### (Method for introducing reactive functional group)

A reactive functional group can be further introduced into the crosslinking agent obtained by the method. Accordingly, a method for producing a protein or peptide in one aspect is a method for producing a protein or peptide where a reactive functional group is bound, the protein or peptide being represented by the following formula: 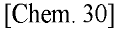 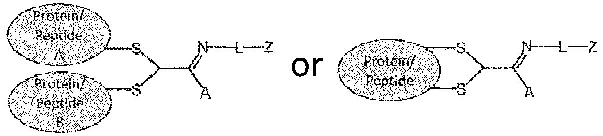 wherein A is a hydrogen atom, a C1 to 6 alkyl group optionally substituted with a phenyl group or a halogen atom, a phenyl group, or a C1 to 6 alkyl group, Protein/Peptide represents a protein or peptide, Protein/Peptide A and Protein/Peptide B are optionally the same or different, L represents a linker and Z represents a reactive functional group;
the method including:
obtaining a crosslinked protein or peptide by the method; and
reacting the crosslinked protein or peptide obtained, with NH₂-L-Z (L represents a linker and Z represents a reactive functional group), to thereby introduce a reactive functional group into a crosslinked portion of the crosslinked protein or peptide.

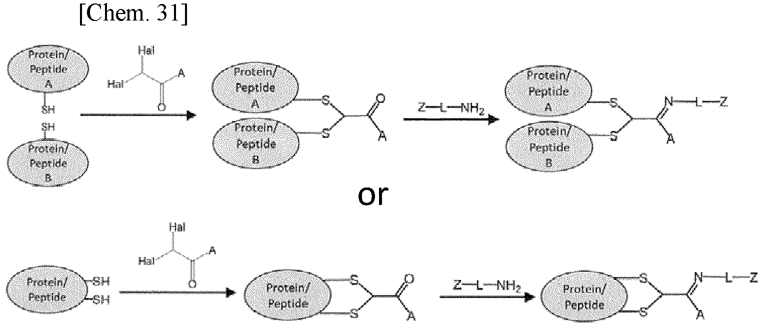

Alternatively, in another aspect, the method for producing a protein or peptide where a reactive functional group is bound can be performed by use of a crosslinked substance. Accordingly, in another aspect, the method may be a method for producing a protein or peptide where a reactive functional group is bound: 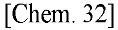 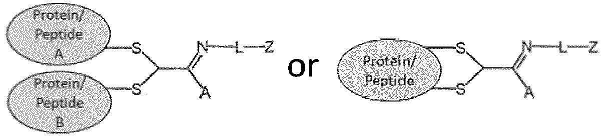
wherein A is a hydrogen atom, a C1 to 6 alkyl group optionally substituted with a phenyl group or a halogen atom, a phenyl group, or a C1 to 6 alkyl group, Protein/Peptide represents a protein or peptide, Protein/Peptide A and Protein/Peptide B are optionally the same or different, L represents a linker and Z represents a reactive functional group;
the method including:
   reacting a crosslinked protein or peptide represented by the following formula: 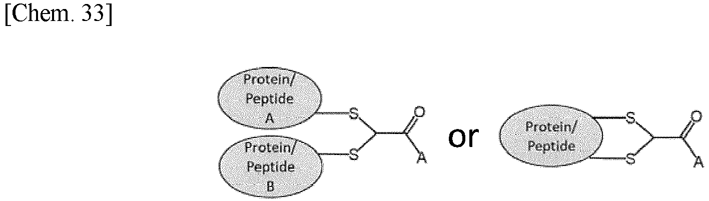
wherein A is a hydrogen atom, a C1 to 6 alkyl group optionally substituted with a phenyl group or a halogen atom, a phenyl group, or a C1 to 6 alkyl group, Protein/Peptide represents a protein or peptide, and Protein/Peptide A and Protein/Peptide B are optionally the same or different;
and NH₂-L-Z (L represents a linker and Z represents a reactive functional group), to thereby introduce a reactive functional group to a crosslinked portion of the crosslinked protein or peptide.

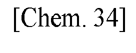

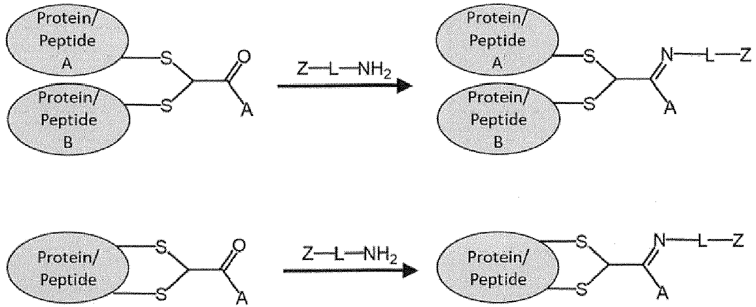

A method for producing a protein or peptide of another aspect is a method for producing a protein or peptide where a reactive functional group is bound, the protein or peptide being represented by the following formula: 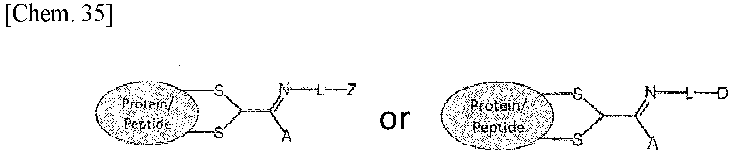
wherein A is a hydrogen atom, a C1 to 6 alkyl group optionally substituted with a phenyl group or a halogen atom, a phenyl group, or a C1 to 6 alkyl group, Protein/Peptide represents a protein or peptide, L represents a linker, Z represents a reactive functional group and D represents a drug;
the method including:
   synthesizing the protein or peptide by an Fmoc method; and
   using a compound represented by the following formula, instead of at least one cysteine residue, in the synthesis. 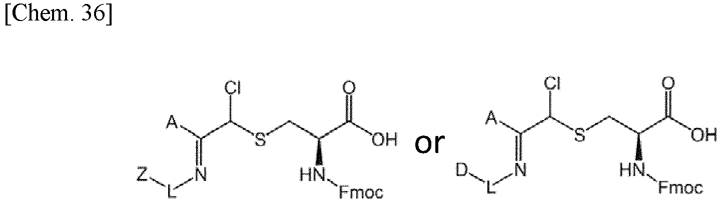
The present method can be performed by the same method as described above (method for producing a crosslinked protein or peptide by peptide synthesis) except that the cysteine residue used is the above compound.

### (Reactive functional group)

Specifically, the reactive functional group can be introduced by reacting a protein or peptide crosslinked by the crosslinking agent according to the present embodiment, and an amino compound (NH₂-L-Z) having the reactive functional group, to thereby form an oxime (oxime method). The amino compound having the reactive functional group, here used, can be NH₂-X-L'-Z (wherein X is O, NH, or N(C1 to 4 alkyl)).

### (Linker)

Herein, L and L' each represent a linker. Herein, the "linker" is not particularly limited as long as it has a length allowing for binding of the reactive functional group or a drug, to be bound to the linker, and the crosslinked protein or peptide and has a structure having no influence on any crosslinking reaction, and may be absent (may represent a bond). As one example, the linker may contain one or more alkylenes, alkenylenes, and alkynylenes. Such alkylenes, alkenylenes, and alkynylenes may be each straight, branched, or cyclic. The number of carbon atoms in the alkylene can be 1 to 20, 1 to 10, 1 to 6, 1 to 4, 1 to 3, 1 to 2, 2, or 1, and the numbers of carbon atoms in the alkenylene and the alkynylene can be each 2 to 20, 2 to 10, 2 to 6, 2 to 4, 2 to 3, or 2. The linker may contain one or more -O-, -S-, -S-S-, -NH-, -N((C1 to C6)alkyl)-, -NH-C(O)-NH-, - C(O)-NH-, -NH-C(O)-, phenylenes, heteroarylenes, and/or heterocyclenes. Furthermore, the linker may contain one or more amino acid moieties, and may be contain, for example, 1 to 100, 1 to 50, 1 to 25, 1 to 20, 1 to 15, 1 to 10, 1 to 5, 1 to 4, 1 to 3, 1 to 2, 2, or 1 amino acid moiety. A hydrogen atom bound to a carbon atom may be replaced by a halogen atom in a group constituting the linker. When an objective is to finally bind the present crosslinking agent to a drug and then release the drug under specified conditions (for example, the present crosslinking agent is intended to be bound (for example, antibody-drug complex (ADC)) to a peptide or protein (for example, an Fc region-containing molecule) directional to a specified target to release a drug at a local rich in the target), the linker contains a moiety cleavable preferably under specified conditions *in vivo,* preferably, conditions characteristic of the local. Examples of such conditions include the presence of an enzyme, and pH. For example, the linker may contain a peptide bond to be cleaved by protease or esterase (for example, GGFG to be cleaved by a lysosome enzyme, see Marcin Poreba, The FEBS Journal (2020) 287: 1936-1969) or an ester. In this case, the linker may contain an amino acid moiety other than the peptide bond to be cleaved by protease.

Herein, the "alkylene" represents a divalent group obtained by removing one hydrogen atom from the alkyl group. Examples of the alkylene group include a C1 to 20 straight or branched alkylene and a C3 to 7 cycloalkylene, and examples thereof include a methylene group, an ethylene group, a propylene group, a butylene group, a pentylene group, a hexylene group, and a cyclohexylene group. The alkylene is, if necessary, optionally substituted with a hydroxyl group, a halogen atom, and an amino group, and in this case, the number of substituents in the alkylene can be 1 to 10, 1 to 8, 1 to 6, 1 to 5, 1 to 4, 1 to 3, 3, 2, and/or 1.

The "alkenylene" means a divalent group obtained by removing two hydrogen atoms from any carbon atom of a straight, branched, or cyclic unsaturated hydrocarbon having one or more carbon-carbon double bonds. Examples of the alkenylene include C2 to 20 alkenylene, and examples thereof include vinylene, propenylene, isopropenylene, butenylene, pentenylene, hexenylene, heptenylene, octenylene, nonenylene, and decenylene.

The "alkynylene" means a divalent group obtained by removing two hydrogen atoms from any carbon atom of a straight or branched unsaturated hydrocarbon having one or more carbon-carbon triple bonds. Examples of the alkynylene include C2 to 20 alkynylene, and examples thereof include ethynylene, propynylene, butynylene, pentynylene, hexynylene, and phenyl ethynylene.

The "heteroarylene" and the "heterocyclene" respectively mean aromatic and non-aromatic 5- to 14-membered divalent monocyclic or fused heterocyclic groups each containing at least one of one or more hetero atoms selected from a nitrogen atom, an oxygen atom and a sulfur atom. The number of hetero atoms contained in the 5- to 14-membered heterocyclic group may be, for example, 1 to 5, 1 to 4, 1 to 3, 1 to 2, 2, or 1. The monocyclic heterocyclic group is preferably a 5- to 6-membered ring. The fused heterocyclic group is preferably an 8- to 10-membered ring. Examples of the heteroarylene and the heterocyclene can include piperidylene, piperazylene, morpholylene, quinuclidylene, pyrrolidylene, azetidylene, oxetylene, aziridinylene, tropanylene, furylene, tetrahydrofurylene, thienylene, pyrrolylene, pyrrolynylene, pyrrolidinylene, dioxolanylene, oxazolylene, oxazolinylene, isoxazolylene, thiazolylene, thiazolinylene, isothiazolylene, imidazolylene, imidazolinylene, imidazolidinylene, oxazolidinylene, thiazolidinylene, pyrazolylene, pyrazolinylene, pyrazolidinylene, oxadiazolylene, furazanylene, thiadiazolylene, triazolylene, tetrazolylene, pyranylene, pyridylene, piperidinylene, pyridazinylene, pyrimidinylene, pyrazinylene, piperazinylene, dioxanylene, oxazinylene, morpholinylene, thiadinylene, triazinylene, benzofuranylene, isobenzofuranylene, dihydrobenzofuranylene, dihydroisobenzofuranylene, benzothienylene, isobenzothienylene, dihydrobenzothienylene, dihydroisobenzothienylene, tetrahydrobenzothienylene, quinolylene, isoquinolylene, quinazolinylene, phthalazinylene, pteridinylene, cumarylene, chromonylene, indolylene, isoindolylene, benzimidazoylene, benzofurylene, purinylene, acridinylene, phenoxazinylene, phenothiazinylene, benzoxazolylene, benzothiazolylene, indazolylene, benzimidazolylene, benzodioxolanylene, benzodioxanyl chromenylene, chromanylene, isochromanylene, chromanonylene, cinnolinylene, quinoxalinylene, indolizinylene, quinolidinylene, imidazopyridylene, naphthylidinylene, dihydrobenzoxazinylene, dihydrobenzoxazolinonylene, dihydrobenzoxazinonylene, and benzothioxanylene.

Herein, the term "including" is designated to mean the inclusion of the case "composed of/consisting of', and can be read as "composed of/consisting of'.

Herein, the "reactive functional group" or the group represented by "Z" means a group capable of reacting and binding with a peptide, a protein, nucleic acid, a low-molecular pharmaceutical, or the like under relatively mild conditions. Examples of the reactive functional group can include maleimide, thiol or protected thiol, alcohol, acrylate, acrylamide, amine or protected amine, carboxylic acid or protected carboxylic acid, azide, alkyne including cycloalkyne, 1,3-diene including cyclopentadiene and furan, alpha-halocarbonyl, N-hydroxysuccinimidyl, N-hydroxysulfosuccinimidyl, nitrophenyl ester, carbonate, dibenzocyclooctyne (DBCO), tetrazine, methyltetrazine (MTZ), trans-cyclooctene (TCO), azide, carboxy, tosyl, amino, epoxy, acyl, isothiocyanate, isocyanate, acyl azide, NHS ester, acid chloride, aldehyde, glyoxal, epoxide, oxirane, carbonate, an arylating agent, imide ester, carbodiimide, acid anhydride, haloacetyl, alkyl halide, maleimide, aziridine, an acryloyl derivative, an arylating agent, diazoalkane, a diazoacetyl compound, carbonyl, ketone, carbodiimide, epoxide, oxirane, carbonyldiimidazole, N,N'-disuccinimidyl carbonate, N-hydroxysuccinimidyl chloroformate, alkyl halide, isocyanate, hydrazine, Schiff base, reductive amination product, a Mannich condensation product, a diazonium derivative, a Mannich condensation product, an iodination reaction product, arylazide, arylazide halide, benzophenone, a diazo compound, and a diaziridine derivative.

The reactive functional group may be a group imparting a covalent bond by an amide bond, a disulfide bond, a thioether bond, a thioester bond, a hydrazone bond, an ester bond, an ether bond, or a urethane bond, in a bound form with a drug. For example, the reactive functional group suitable for reaction with primary amine is N-succinimidyl ester or N-sulfosuccinimidyl ester, the reactive functional group suitable for reaction with an amino group is p-nitrophenyl ester, dinitrophenyl ester or pentafluorophenyl ester, the reactive functional group suitable for reaction with a mercapto group is a maleimide group, carboxylic acid chloride, a pyridyldithio group, a nitropyridyldithio group, a haloalkyl group, and a haloacetyl group, and the reactive functional group suitable for reaction with a hydroxy group is an isocyanate group (isocyanate) (Greg t. Hermanson, Bioconjugate Techiniques Second Edition, p 234-345).

The reactive functional group further encompasses also a reactive functional group involving in (capable of) reaction that forms an oxime bond by reaction with alkoxyamine, Huisgen's 1,3- dipolar cyclization addition reaction ("Click" reaction) that is reaction with alkyne or azide by a Cu(I) catalyst, inverse-electron-demand Diels-Alder reaction, Michael reaction, metathesis reaction, cross-coupling reaction by a transition metal catalyst, radical polymerization reaction, oxidative-coupling reaction, transacylation reaction, or photoclick reaction (Kim CH et al, Curr Opin Chem Biol. 2013 Jun; 17(3):412-9).

As one example, reactive functional group introduction can be performed by introducing a reactive functional group such as an alkyne group, due to reaction of the protein or the peptide or its fused product bound via the crosslinking agent, and a hydroxyamine derivative, in a buffer such as PBS (pH 7.4) under an environment at 4°C to ordinary temperature for 30 minutes to overnight.

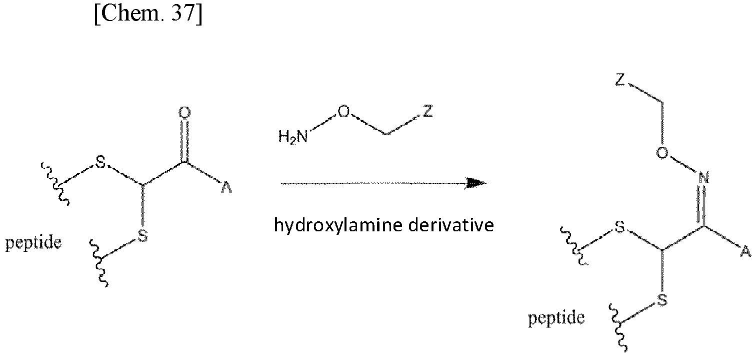

### (Drug binding method)

The crosslinking agent according to the present embodiment can be utilized to thereby introduce a drug (payload) into a protein or peptide or its fused product. Specifically, a reactive functional group of a protein or peptide or its fused product where the reactive functional group is introduced, and a drug (payload) can be reacted and bound to thereby introduce the drug (payload) into the protein or peptide or its fused product.

Accordingly, in one aspect, a method for producing a complex of a protein or peptide and a drug is a method for producing a complex of a protein or peptide and a drug, the complex being represented by the following formula: 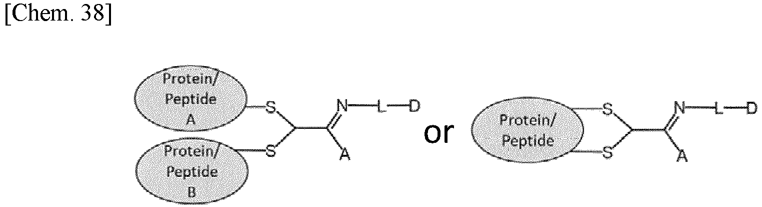
wherein A is a hydrogen atom, a C1 to 6 alkyl group optionally substituted with a phenyl group or a halogen atom, a phenyl group, or a C1 to 6 alkyl group, Protein/Peptide represents a protein or peptide, Protein/Peptide A and Protein/Peptide B are optionally the same or different, L represents a linker and D represents a drug;
the method comprising:
   obtaining a protein or peptide where a reactive functional group is bound, by the method; and
   reacting the obtained protein or peptide where a reactive functional group is bound, and a drug having a functional group reactive with the reactive functional group, to thereby bind the drug to a crosslinked portion of the crosslinked protein or peptide.

Alternatively, the method for producing a complex of a protein or peptide and a drug can be produced by use of a protein and/or peptide where a reactive functional group is introduced. Accordingly, a method for producing a complex of a protein or peptide and a drug in another aspect is a method for producing a complex of a protein or peptide and a drug, the complex being represented by the following formula: 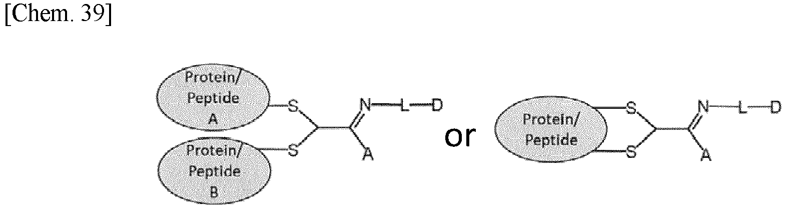
wherein A is a hydrogen atom, a C1 to 6 alkyl group optionally substituted with a phenyl group or a halogen atom, a phenyl group, or a C1 to 6 alkyl group, Protein/Peptide represents a protein or peptide, Protein/Peptide A and Protein/Peptide B are optionally the same or different, L represents a linker and D represents a drug;
the method including:
   reacting a protein or peptide where a reactive functional group is bound, the protein or peptide being represented by the following formula: 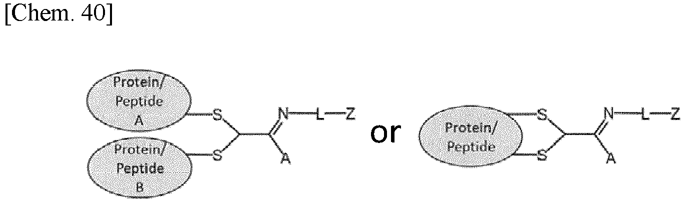
wherein A is a hydrogen atom, a C1 to 6 alkyl group optionally substituted with a phenyl group or a halogen atom, a phenyl group, or a C1 to 6 alkyl group, Protein/Peptide represents a protein or peptide, Protein/Peptide A and Protein/Peptide B are optionally the same or different, L represents a linker and Z represents a reactive functional group; and a drug having a functional group reactive with the reactive functional group, to thereby bind the drug to a crosslinked portion of the crosslinked protein or peptide.

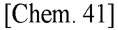

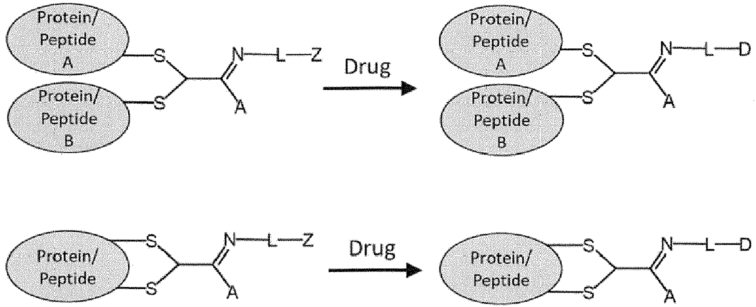

Herein, the "drug" is not particularly limited as long as it is a drug to be introduced into a protein or peptide and then utilized, and examples thereof include a therapeutic agent, a prophylactic agent, a targeting agent, a labeling agent, or a diagnostic agent. Examples of the therapeutic agent or the prophylactic agent can include anticancer agents such as monomethyl auristatin, auristatin, maytansine, emtansine, doxorubicin, bleomycin, ozogamicin, vedotin, pasudotox, deruxtecan, maytansinol calicheamicin, exatecan, a pyrrolobenzodiazepine dimer, duocarmycin, eribulin, SN-38, PNU-159682, emtansine (DM1), mertansine or derivatives thereof; radioisotopes such as ⁹⁰Y; targeting agents such as a drug capable of binding to a receptor on the blood-brain barrier and then transferring to the central nerve and a drug capable of binding to cancer cells and then transferring antibodies into cells; and detectable labels such as a radioactive label, an enzyme, a fluorescent label, a bioluminescent label, and a chemiluminescent label metal.

As one example, the drug can be introduced by reaction of a protein or peptide or its fused product where a maleimide group as the reactive functional group is introduced by oxime reaction in advance, with one obtained by introduction of an azide group to an SH group of mertansine being an anticancer agent, as the drug, in a buffer such as PBS (pH 7.4) under an environment at 4°C to ordinary temperature for 30 minutes to overnight.

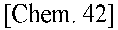

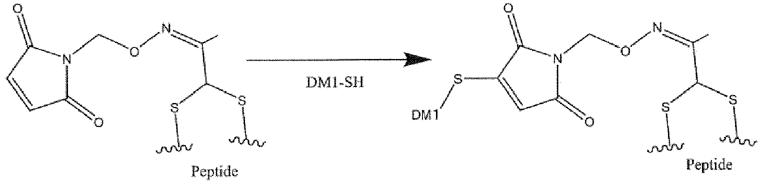

### (Crosslinked protein or peptide)

One aspect provides crosslinked protein and/or peptide by the crosslinking agent. Another aspect provides a fused protein or fused peptide having the following structure, in which two thiol groups present in separated protein and/or peptide are bound to each other via the crosslinking agent. Hereinafter, Protein/Peptide A and Protein/Peptide B are optionally the same substance as or different substances from each other.

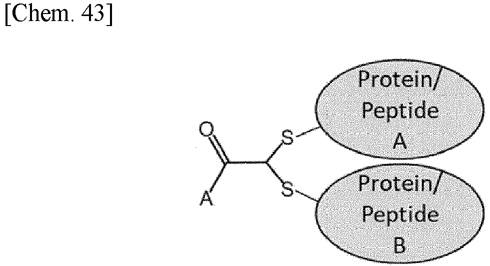

In the formula, A is defined as in formula (I).

Another aspect provides a protein or peptide having the following structure where two thiol groups present in a single protein or peptide are bound to each other via the crosslinking agent of the present disclosure.

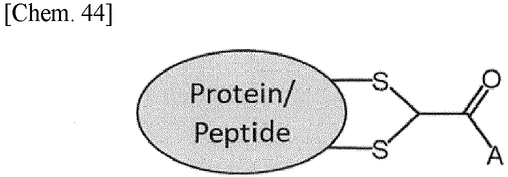

In the formula, A is defined as in formula (I).

The crosslinking agent according to the present embodiment can be bound with the reactive functional group or the drug to thereby introduce the functional group or the drug to a desired protein, peptide, or complex thereof. Accordingly, the crosslinked protein and peptide may be a protein or peptide where the reactive functional group is bound via the crosslinking agent, or may a protein or peptide where the drug is bound via the crosslinking agent, and has a molecule represented below.

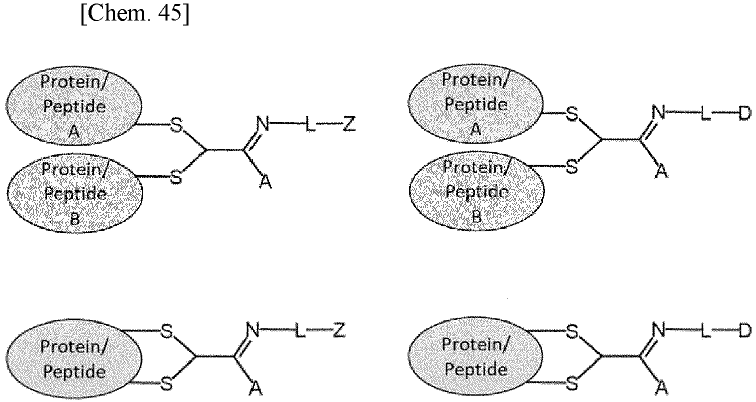

In the formula, L represents the linker, Z represents the reactive functional group, and D represents the drug.

Herein, the linker can have a branched structure to thereby bind with a plurality of functional groups Z or drugs D. The number of functional groups Z or drugs D bound to the crosslinking agent according to the present embodiment can be 1 to 20, 1 to 10, 1 to 8, 1 to 7, 1 to 6, 1 to 5, 1 to 4, 1 to 3, 1 to 2, or 1. For example, when the number of functional groups Z or drugs D is 2, the following structure can be adopted (L1 and L2 each represent a linker; The linker is defined as described above.).

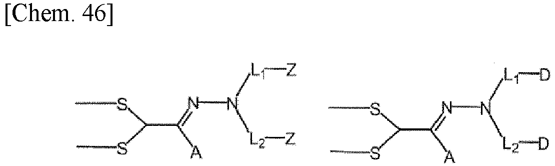

The crosslinking agent according to the present embodiment can be used for the purpose of an increase in binding stability of a desired protein, peptide, or complex thereof. For example, in the crosslinked protein or peptide, the group A may be a hydrogen atom, and a molecule represented below is contained.

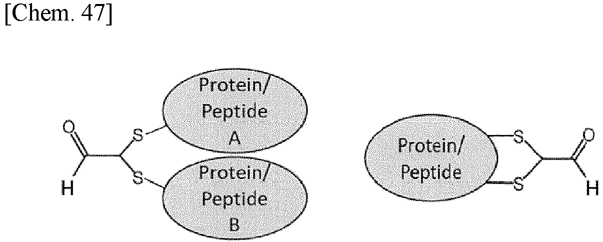

In the above, the linker L, the reactive functional group Z, and the drug D are the same as described above. The molecule crosslinked is a molecule improved in alkali resistance and stabilized as compared with a disulfide bond. The number of such binding sites via the crosslinking agent may be one, or two or more. When two or more such crosslinking sites are present, crosslinking in a single protein or peptide may be made, crosslinking in separated protein and/or peptide may be made, or crosslinking in a single protein or peptide and crosslinking in separated protein and/or peptide may be combined. When two or more such crosslinking sites in a separated protein or peptide are present, crosslinking between the same two proteins and/or peptides may be made, or binding by crosslinking among three or more proteins and/or peptides by crosslinking between a plurality of proteins and/or peptides may be made.

### (Crosslinked Fc-binding peptide)

The peptide to be crosslinked herein may be an Fc-binding peptide. Accordingly, another aspect provides an Fc-binding peptide represented by the following formula (wherein A, L, Z, and D are defined as described above) and crosslinked via the crosslinking agent, or a production method thereof.

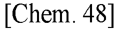

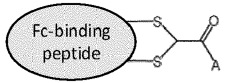

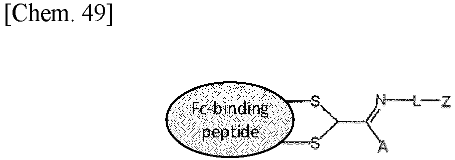

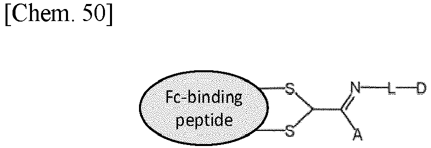

Herein, the "Fc-binding peptide" means a peptide to be specifically bound to an Fc region of IgG. Herein, the "Fc region of IgG" typically means a fragment at the C-terminal, obtained as a product formed by treatment of IgG with papain being a proteolytic enzyme. The Fc-binding peptide is preferably a peptide to be bound to a site selected from Lys248, Lys246, Lys338, Lys288, Lys290, Lys360, Lys414, and Lys439 of Fc according to the Eu numbering, and/or an adjacent region thereof, preferably Lys248 and/or an adjacent region thereof, or to be bound to a binding region of protein A. For example, the Fc-binding peptide may be a partial peptide of protein A having an Fc-binding ability, or a mutant thereof. Specific examples of such a peptide are described in International Publication No. WO 2008/054030, International Publication No. WO 2013/027796, International Publication No. WO 2016/186206, International Publication No. WO 2018/230257, and Kyohei Muguruma et al., ACS Omega (2019); 4(11): 14390-14397, and such a peptide can be appropriately prepared according to a method described in each of these Documents. In the Fc-binding peptide, preferably, two cysteine residues, cysteine closest to the C-terminal and cysteine closest to the N-terminal, are crosslinked via the crosslinking agent herein.

Herein, the "IgG" may be IgG from mammals, for example, primates such as humans and chimpanzees, lab animals such as rats, mice, and rabbits, livestock animals such as pigs, cattle, horses, sheep, and goats, and pet animals such as dogs and cats, and is preferably human IgG (IgG1, IgG2, IgG3 or IgG4). Herein, the IgG is preferably human IgG1, IgG2, or IgG4, or rabbit IgG, and is particularly preferably human IgG1, IgG2, or IgG4.

Specifically, the Fc-binding peptide may be a peptide selected from the following (i) to (iv):
(i) a peptide represented by the following formula (I):

   NH₂-(Linker)-(X¹₁₋₃)-C-(X²)-(X³)-(X⁴)-(X⁵)-G-(X⁶)-L-(X⁷)-W-C-(X⁸₁₋₃) ... (I)

   wherein (Linker) represents a linker, one to three X¹s, X²s, X³s, X⁴s, X⁵s, X⁶s, and X⁷s, and one to three X⁸s each independently represent the same or different amino acid residues,
   each of X¹, X², and X³, and each X⁸ independently represent any amino acid residues other than C, which are the same or different,
   X⁴ is H, R, S, or D,
   X⁵ is one amino acid residue selected from K, C, D, E, R, V, F, L, 2-aminosuberic acid, Dpr, Orn, AcOrn, AcDab, Dab, Nle, Nva, Tle, Ala(t-Bu), and Cha,
   X⁶ is E, N, R, or D, and
   X⁷ is I or V;
(ii) a peptide represented by the following formula (II), or a peptide containing an amino acid sequence of (II), where one or several amino acids are added, deleted and/or substituted at a position other than X⁹ to X¹³:

   X⁹₁₋₂NMQCQRRFYEALHDPNLNEEQRNAX¹¹IX¹²SIRDDC-(Linker 2)-CONH₂(SEQ ID NO: 2) ... (II)

   wherein (Linker 2) represents a linker, X⁹₁₋₂ is selected from the group consisting of GF, AF, VF, LF, IF, MF, PF, FF, WF, KF, Om-F, CF, DF, EF, (βAla-F, 2-aminosuberic acid-F, Dpr-F, and NH₂-(PEG)ₙ-CO (n = 1 to 50)-F, F, K, Orn, C, D, E, 2-aminosuberic acid residue, and Dpr, and
   X¹¹ and X¹² are each independently selected from the group consisting of R, H, D, E, S, T, N, Q, Y, and C;
(iii) a peptide represented by the following formula (I') or (I"):

   Z-[(Linker3)-(X¹₁₋₃)-C-(X²)-(X³)-(X⁴)-(X⁵)-G-(X⁶)-L-(X⁷)-W-C-(X⁸₁₋₃)] ... (I')

   [(X¹₁₋₃)-C-(X²)-(X³)-(X⁴)-(X⁵)-G-(X⁶)-L-(X⁷)-W-C-(X⁸₁₋₃)-(Linker3)]-Z ... (I")

   wherein
   Z represents a reactive functional group,
   (Linker3) represents a linker,
   one to three X¹s, X²s, X³s, X⁴s, X⁵s, X⁶s, and X⁷s, and one to three X⁸s each independently represent the same or different amino acid residues,
   each of X¹, X², and X³, and each X⁸ independently represent any amino acid residues other than C, which are the same or different,
   X⁴ is H, R, S, or D,
   X⁵ is one amino acid residue selected from K, C, D, E, R, V, F, L, 2-aminosuberic acid, Dpr, Orn, AcOrn, AcDab, Dab, N1e, Nva, Tle, Ala(t-Bu), and Cha,
   X⁶ is E, N, R, or D, and
   X⁷ is I or V;
(iv) a peptide containing an amino acid sequence represented by the following formula (II'), or a peptide containing an amino acid sequence of (II'), where one or several amino acids are added, deleted and/or substituted at a position other than X⁹ to X¹⁴:

   X⁹₁₋₂NMQCQX¹⁴RFYEALHDPNLNEEQRNAX¹¹IX¹²SIRDDC-(Linker 2)-NH₂ (SEQ ID NO: 3) ... (II')

   wherein (Linker 2) represents a linker, X⁹₁₋₂ is selected from the group consisting of GF, AF, VF, LF, IF, MF, PF, FF, WF, KF, Om-F, CF, DF, EF, (βAla-F, 2-aminosuberic acid-F, Dpr-F, and NH₂-(PEG)ₙ-CO (n = 1 to 50)-F, F, K, Orn, C, D, E, 2-aminosuberic acid residue, Dpr, and Acetyl-K,
   X¹¹ and X¹² are each independently selected from the group consisting of R, H, D, E, S, T, N, Q, Y, C, and K(Z),
   X¹⁴ is R, C, K(Z), and
   Z is a reactive functional group.

Herein, X^{m} (m is an integer) represents an amino acid. "X^{m}ₙ" represents binding of n of amino acids X^{m}, and "X^{m}" where no n is designated represents the presence of one amino acid X^{m}. When n is 2 or more, a plurality of X^{m} may be each independently the same or different amino acids. A case where n is "p-q" represents the presence of p to q of amino acids X^{m}. In the amino acid sequences described herein, A is an alanine residue, R is an arginine residue, N is an asparagine residue, D is an aspartic acid residue, C is a cysteine residue, Q is a glutamine residue, E is a glutamic acid residue, G is a glycine residue, H is a histidine residue, I is an isoleucine residue, L is a leucine residue, K is a lysine residue, M is a methionine residue, F is a phenylalanine residue, P is a proline residue, S is a serine residue, T is a threonine residue, W is a tryptophan residue, Y is a tyrosine residue, and V is a valine residue. Hcy is homocysteine, Dpr is diaminopropionic acid, Orn is an ornithine residue, (βAla is a β-alanine residue, Dab is a 2,4-diaminobutyric acid residue, Nle is a norleucine residue, Nva is a norvaline residue, Tle is a tert-leucine residue, Ala(t-Bu) is a tert-butylalanine residue, and Cha is a cyclohexylalanine residue. In a residue (lysine residue, ornithine residue, 2,4-diaminobutyric acid residue) containing an amino group at a side chain, the amino group may be, if necessary, acetylated. Herein, each acetylated form of natural and artificial amino acids may also be designated with the prefix Ac in the designation of such an amino acid, and such an amino acid is interpreted to be capable of encompassing such an acetylated form even if not designated with Ac, unless such an interpretation is particularly inconsistent. Herein, K(Z) means a functional group-bound lysine residue, and K(Z) is preferably K(Azide). K(Azide) represents an azide-bound lysine residue.

Herein, the Fc-binding peptide may be a peptide for carrier-binding, which is for binding with a carrier to thereby bind an antibody and the carrier, or may be a peptide for drug-binding, which is for binding a drug to an antibody via the peptide. In the case of the peptide for carrier-binding, the peptide (i) or (ii) is preferable.

In formula (I), Linker is (GSGGS)₁₋₃, (SGSGS)₁₋₃, (GGGGS)₁₋₃, or (PEG)₂₋₁₀ (preferably, (PEG)₄), or absent. For binding with a carrier, an amino group may be bound to the carboxyl terminal (-COOH) at the C-terminal of formula (I) to provide a (-C(=O)NH₂) group, or Linker (defined as described above) may be arbitrarily inserted between the carboxyl terminal and an amino group. When Linker is present at the C-terminal, no Linker at the N-terminal side may be present. In other words, (X¹₁₋₃)-C-(X²)-(X³)-(X⁴)-(X⁵)-G-(X⁶)-L-(X⁷)-W-C-(X⁸₁₋₃)-(Linker)-NH₂ may also be formed. The amino group at the N-terminal of formula (I) may be acetylated (in this case, a Lys residue is introduced to an appropriate position in the vicinity of the N-terminal in Linker at the N-terminal side).

Examples of the peptide represented by formula (I) preferably include the following peptides.
[1] X¹₁₋₃ is an amino acid sequence represented by (S, G, F, or absent)-(D, G, A, S, P, Hcy, or absent)-(S, D, T, N, E, or R).
[2] X¹₁₋₃ is D, GPD, R, GPR, SPD, GDD, GPS, SDD, RGN, G-Hcy-D, RGP, or GPD.
[3] X¹₁₋₃ is D or GPD.
[4] X² is A, S, or T.
[5] X² is A or T.
[6] X² is A.
[7] X³ is Y or W.
[8] X³ is Y.
[9] X⁴ is H.
[10] X⁵ is one amino acid residue selected from A, R, K, C, D, E, L, 2-aminosuberic acid, Dpr, R, F, 2-aminosuberic acid, Dpr, AcOrn, AcDab, Dab, Nle, Nva, Ala(t-Bu), and Cha.
[11] X⁵ is K, R, or AcOrn.
[12] X⁵ is one amino acid residue selected from V, Dab, F, R, L, Nva, Nle, Ala(t-Bu), and Cha.
[13] X⁵ is one amino acid residue selected from F, R, L, Nva, Nle, Ala(t-Bu), and Cha.
[14] X⁵ is one amino acid residue selected from L, Ala(t-Bu), and Cha.
[15] X⁶ is E orN.
[16] X⁶ is E.
[17] X⁷ is V.
[18] X⁸₁₋₃ is (S, T, or D)-(H, G, Y, T, N, D, F, Hcy, or absent)-(Y, F, H, M, or absent).
[19] X⁸₁₋₃ is T, TFH, S, SFH, THH, TFY, TYH, or T-Hcy-H.
[20] X⁸₁₋₃ is T or TFH.

The peptide represented by formula (I) may be a peptide satisfying any one, or a combination of two or more of the above conditions, or may be, for example, a peptide satisfying the following conditions: [8] and [9]; [8] and [17]; [9] and [17]; [8] and [9] and [17]; or a combination thereof with any one of [10] to [14].

More specifically, examples thereof can include the following peptides (hereinafter, X⁵ is the same as described above, an NH₂-(Linker)-group may be present at the N-terminal, and an -NH₂ group or an NH₂-(Linker)-group may be present at the C-terminal):
1) DCAYHX⁵GELVWCT (SEQ ID NO: 4)
2) GPDCAYHX^{s}GEL VWCTFH (SEQ ID NO: 5)
3) RCAYHX⁵GELVWCS (SEQ ID NO: 6)
4) GPRCAYHX⁵GELVWCSFH (SEQ ID NO: 7)
5) SPDCAYHX⁵GELVWCTFH (SEQ ID NO: 8)
6) GDDCAYHX⁵GELVWCTFH (SEQ ID NO: 9)
7) GPSCAYHX⁵GELVWCTFH (SEQ ID NO: 10)
8) GPDCAYHX⁵GELVWCSFH (SEQ ID NO: 11)
9) GPDCAYHX⁵GELVWCTHH (SEQ ID NO: 12)
10) GPDCAYHX⁵GELVWCTFY (SEQ ID NO: 13)
11) SPDCAYHX⁵GELVWCTFY (SEQ ID NO: 14)
12) SDDCAYHX⁵GELVWCTFY (SEQ ID NO: 15)
13) RGNCAYHX⁵GQLVWCTYH (SEQ ID NO: 16)
14) G-Hcy-DCAYHX⁵GELVWCT-Hcy-H (SEQ ID NO: 17)
15) RRGPDCAYHX^{s}GELVWCTFH (SEQ ID NO: 18)
16) DCTYHX⁵GNLVWCT (SEQ ID NO: 19)
17) DCAYHX⁵GNLVWCT (SEQ ID NO: 20)
18) DCTYHX⁵GELVWCT (SEQ ID NO: 21)
19) DCAWHX⁵GELVWCT (SEQ ID NO: 22)
20) DCTYTX⁵GNLVWCT (SEQ ID NO: 23)
21) DCAYTX⁵GNLVWCT (SEQ ID NO: 24)
22) DCSYTX⁵GNLVWCT (SEQ ID NO: 25)
23) DCTWTX⁵GNLVWCT (SEQ ID NO: 26)
24) DCTYHX⁵GNLVWCT (SEQ ID NO: 27)
25) DCTYRX⁵GNLVWCT (SEQ ID NO: 28)
26) DCTYSX⁵GNLVWCT (SEQ ID NO: 29)
27) DCTYTX⁵GNLVWCT (SEQ ID NO: 30)
28) DCTYTX⁵GELVWCT (SEQ ID NO: 31)
29) DCTYTX⁵GRLVWCT (SEQ ID NO: 32)
30) DCTYTX⁵GDLVWCT (SEQ ID NO: 33)
31) DCTYTX⁵GNLIWCT (SEQ ID NO: 34)
32) DCAYHRGELVWCT (SEQ ID NO: 35)
33) GPDCAYHRGELVWCTFH (SEQ ID NO: 36)
34) RCAYHRGELVWCS (SEQ ID NO: 37)
35) GPRCAYHRGELVWCSFH (SEQ ID NO: 38)
36) SPDCAYHRGELVWCTFH (SEQ ID NO: 39)
37) GDDCAYHRGELVWCTFH (SEQ ID NO: 40)
38) GPSCAYHRGELVWCTFH (SEQ ID NO: 41)
39) GPDCAYHRGELVWCSFH (SEQ ID NO: 42)
40) GPDCA YHRGEL VWCTHH (SEQ ID NO: 43)
41) GPDCAYHRGELVWCTFY (SEQ ID NO: 44)
42) SPDCAYHRGELVWCTFY (SEQ ID NO: 45)
43) SDDCAYHRGELVWCTFY (SEQ ID NO: 46)
44) DCTYHRGNLVWCT (SEQ ID NO: 47)
45) DCAYHRGNLVWCT (SEQ ID NO: 48)
46) DCTYHRGELVWCT (SEQ ID NO: 49)
47) DCAWHRGELVWCT (SEQ ID NO: 50)
48) DCTYTNGNLVWCT (SEQ ID NO: 51)
49) DCAYTNGNLVWCT (SEQ ID NO: 52)
50) DCSYTNGNLVWCT (SEQ ID NO: 53)
51) DCTWTNGNLVWCT (SEQ ID NO: 54)
52) DCTYHNGNLVWCT (SEQ ID NO: 55)
53) DCTYRNGNLVWCT (SEQ ID NO: 56)
54) DCTYSNGNLVWCT (SEQ ID NO: 57)
55) DCTYTRGNLVWCT (SEQ ID NO: 58)
56) DCTYTNGELVWCT (SEQ ID NO: 59)
57) DCTYTNGRLVWCT (SEQ ID NO: 60)
58) DCTYTNGDLVWCT (SEQ ID NO: 61)
59) DCTYTNGNLIWCT (SEQ ID NO: 62)

As one example, a peptide having the following structure can be used for carrier binding.

GSGGS-GPDCAYHRGELVWCTFH-NH₂

(PEG)₄-GPDCAYHRGELVWCTFH-NH₂

GSGGS-DCAYHRGELVWCT-NH₂

(PEG)₄-DCAYHRGELVWCT-NH₂

In formula (II), Linker 2 is (GSGGS)₁₋₃, (SGSGS)₁₋₃, (GGGGS)₁₋₃ or (PEG)₂₋₁₀-Lys (preferably, PEG)₄-Lys), or absent. The terminal (-NH²) of the N-terminal amino acid of formula (II) may be acetylated and thus in the form of a (CH₃-C(=O)-NH-) group. Linker 2 may be bound to the amino terminal (in this case, a Lys residue is introduced to an appropriate position in the vicinity of the N-terminal in Linker at the N-terminal side), and in this case, Linker 2 at the C-terminal may be present or absent.

Examples of the peptide containing the amino acid sequence represented by formula (II) preferably include the following peptides.
[21] X⁹ is selected from the group consisting of GF, AF, βAlaF, NH₂-(PEG)ₙ-CO (n = 2 to 10)-F, F, K, Orn, C, and Dpr.
[22] X⁹ is selected from the group consisting of GF, F, and K.
[23] X¹¹ and X¹² are each independently selected from the group consisting of R, H, and E.
[24] X¹¹ and X¹² are each R.

Examples of the peptide containing the amino acid sequence represented by formula (II) more specifically can include the following peptides:
60) FNMQCQRRFYEALHDPNLNEEQRNARIRSIRDDC (SEQ ID NO: 63),
61) GFNMQCQRRFYEALHDPNLNEEQRNARIRSIRDDC (SEQ ID NO: 64),
62) KNMQCQRRFYEALHDPNLNEEQRNARIRSIRDDC (SEQ ID NO: 65),
63) GFNMQCQKRFYEALHDPNLNEEQRNARIRSIRDDC (SEQ ID NO: 66),
64) KNMQCQKRFYEALHDPNLNEEQRNARIRSIRDDC (SEQ ID NO: 67),
or
66) GKNMQCQRRFYEALHDPNLNEEQRNARIRSIRDDC (SEQ ID NO: 68).

For example, a peptide having the following structure can be used as the peptide for carrier-binding.
Acetyl-FNMQCQRRFYEALHDPNLNEEQRNARIRSIRDDC-SGSGSK-NH₂
Acetyl-FNMQCQRRFYEALHDPNLNEEQRNARIRSIRDDC-(PEG)₄-Lys-NH₂

The peptide for carrier-binding has at least one amino group (-NH²) for a covalent bond with a carrier. Such an amino group is preferably an amino group at the N-terminal, and may be any side-chain amino group of a lysine residue, a cysteine residue, an aspartic acid residue, a glutamic acid residue, a 2-aminosuberic acid, Dpr, and an arginine residue in the vicinity of the N-terminal or the C-terminal (for example, positioned in the linker) as long as binding with a carrier can be made.

In the case of the peptide for drug-binding, the peptide (iii) or (iv) is preferable.

The peptide represented by formula (I') may contain a functional group at the C-terminal, instead of a functional group at the N-terminal. In other words, the peptide represented by formula (I') may be a peptide represented by the following formula (I"):

[(X¹₁₋₃)-C-(X²)-(X³)-(X⁴)-(X⁵)-G-(X⁶)-L-(X⁷)-W-C-(X⁸₁₋₃)-(Linker3)]-Z ... (I")

wherein Z represents a functional group, and is bound to any moiety of a structure represented by [(X¹₁₋₃)-C-(X²)-(X³)-(X⁴)-(X⁵)-G-(X⁶)-L-(X⁷)-W-C-(X⁸₁₋₃)-(Linker3)], (Linker3) represents a linker, and one to three X¹s, X²s, X³s, X⁴s, X⁵s, X⁶s, and X⁷s, and one to three X⁸s each independently represent the same or different amino acid residues,
each of X¹, X², and X³, and each X⁸ independently represent any amino acid residues other than C, which are the same or different,
X⁴ is H, R, S, or T,
X⁵ is one amino acid residue selected from K, C, D, E, R, V, F, L, 2-aminosuberic acid, Dpr, Orn, AcOrn, AcDab, Dab, N1e, Nva, Tle, Ala(t-Bu), and Cha,
X⁶ is E, N, R, or D, and
X⁷ is I or V.

In formula (I') and formula (I"), Linker 3 is RRRGS, EEGGS or (PEG)₁₋₈ (preferably, (PEG)₄), or absent. An amino group may be bound to the terminal (-COOH) of the C-terminal amino acid of formula (I') and thus in the form of a (-C(=O)NH₂) group. An acetyl group may be bound to the terminal (-NH²) of the N-terminal amino acid of formula (I") and thus in the form of a (CH₃-C(=O)-NH-) group.

The peptides having the amino acid sequences represented by formula (I') and formula (I") may be each Z-(Linker3)-(X¹₁₋₃)-C-(X²)-(X³)-(X⁴)-(X⁵)-G-(X⁶)-L-(X⁷)-W-C-(X⁸₁₋₃) or (X¹₁₋₃)-C-(X²)-(X³)-(X⁴)-(X⁵)-G-(X⁶)-L-(X⁷)-W-C-(X⁸₁₋₃)-(Linker3)-Z. A preferable amino acid sequence is the same as a preferable amino acid sequence in the peptide represented by formula (I).

Examples of the peptide for drug-binding can include the following peptides.
Acetyl-K(Z)-RRRGS-GPDCAYHKGELVWCTFH-NH₂
Acetyl-K(Z)-EEGGS-GPDCAYHKGELVWCTFH-NH₂
Acetyl-K(Z)-(PEG)₄-GPDCAYHKGELVWCTFH-NH₂
Maleimide-RRRGS-GPDCAYHKGELVWCTFH-NH₂
Maleimide-EEGGS-GPDCAYHKGELVWCTFH-NH₂
Maleimide-(PEG)₄-GPDCAYHKGELVWCTFH-NH₂
DBCO-RRRGS-GPDCAYHKGELVWCTFH-NH₂
DBCO-EEGGS-GPDCAYHKGELVWCTFH-NH₂
DBCO-(PEG)₄-GPDCAYHKGELVWCTFH-NH₂
Tetrazine-RRRGS-GPDCAYHKGELVWCTFH-NH₂
Tetrazine-EEGGS-GPDCAYHKGELVWCTFH-NH₂
Tetrazine-(PEG)₄-GPDCAYHKGELVWCTFH-NH₂
TCO-RRRGS-GPDCAYHKGELVWCTFH-NH₂
TCO-EEGGS-GPDCAYHKGELVWCTFH-NH₂
TCO-(PEG)₄-GPDCAYHKGELVWCTFH-NH₂
Acetyl-K(Z)RRRGS-DCAYHKGELVWCT-NH₂
Acetyl-K(Z)EEGGS-DCAYHKGELVWCT-NH₂
Acetyl-K(Z)-(PEG)₄-DCAYHKGELVWCT-NH₂
Maleimide-RRRGS-DCAYHKGELVWCT-NH₂
Maleimide-EEGGS-DCAYHKGELVWCT-NH₂
Maleimide-(PEG)₄-DCAYHKGELVWCT-NH₂
DBCO-RRRGS-DCAYHKGELVWCT-NH₂
DBCO-EEGGS-DCAYHKGELVWCT-NH₂
DBCO-(PEG)₄-DCAYHKGELVWCT-NH₂
Tetrazine-RRRGS-DCAYHKGELVWCT-NH₂
Tetrazine-EEGGS-DCAYHKGELVWCT-NH₂
Tetrazine-(PEG)₄-DCAYHKGELVWCT-NH₂
TCO-RRRGS-DCAYHKGELVWCT-NH₂
TCO-EEGGS-DCAYHKGELVWCT-NH₂
TCO-(PEG)₄-DCAYHKGELVWCT-NH₂

In formula (II'), Linker 2 is SGSGSK, SRRCR, SRRK(Z)R, SRRCRRCRRC, SRRK(Z)RRK(Z)RRK(Z), or (PEG)₁₋₈-Lys (preferably, (PEG)₄-Lys), or absent. The terminal (-NH²) of the N-terminal amino acid of formula (II') may be acetylated and thus in the form of a (CH₃-C(=O)-NH-) group. Other functional molecule may also be, if necessary, bound to a Cys residue (C) contained in the linker via a maleimide group.

Examples of the peptide containing the amino acid sequence represented by formula (II') preferably include the following peptides.
[a] X⁹ is selected from the group consisting of GF, AF, βAlaF, NH₂-(PEG)ₙ-CO (n = 1 to 50)-F, F, K, Orn, C, Dpr, and Acetyl-K.
[b] X⁹ is selected from the group consisting of GF, F, and Acetyl-K.
[b] X¹¹ and X¹² are each independently selected from the group consisting of R, H, and E.
[c] X¹¹ is R.
[d] X¹² is R or K(Z) (preferably, Z is azide).

Examples of the peptide containing the amino acid sequence represented by formula (II') more specifically can include the peptides described in 60) to 66) (where a functional group may be, if necessary, bound to a lysine residue contained):

FNMQQQCRFYEALHDPNLNEEQRNARICSIRDDP-SRRCRRCRRC-NH2
ACetyl-KNMQQQCRFYEALHDPNLNEEQRNARICSIRDDP-SRRCRRCRRC-NH2
GFNMQQQCRFYEALHDPNLNEEQRNARICSIRDDP-SRRCRRCRRC-NH2
FNMQCQZRFYEALHDPNLNEEQRNARIRSIRDDC-NH2
ACetyl-KNMQCQZRFYEALHDPNLNEEQRNARIRSIRDDC-NH2
GFNMQCQK(Z)RFYEALHDPNLNEEQRNARIRSIRDDC-SRRK(Z)R-NH2
FNMQCQK(Z)RFYEALHDPNLNEEQRNARIRSIRDDC-NH2
ACetyl-KNMQCQK(Z)RFYEALHDPNLNEEQRNARIRSIRDDC-SRRK(Z)R-NH2
GFNMQCQK(Z)RFYEALHDPNLNEEQRNARIRSIRDDC-
SRRK(Z)RRK(Z)RRK(Z)-NH2
ACetyl-KNMQCQK(Z)RFYEALHDPNLNEEQRNARIRSIRDDC-
SRRK(Z)RRK(Z)RRK(Z)-NH2
GFNMQCQK(Z)RFYEALHDPNLNEEQRNARIRSIRDDC-
SRRK(Z)RRK(Z)RRK(Z)-NH2

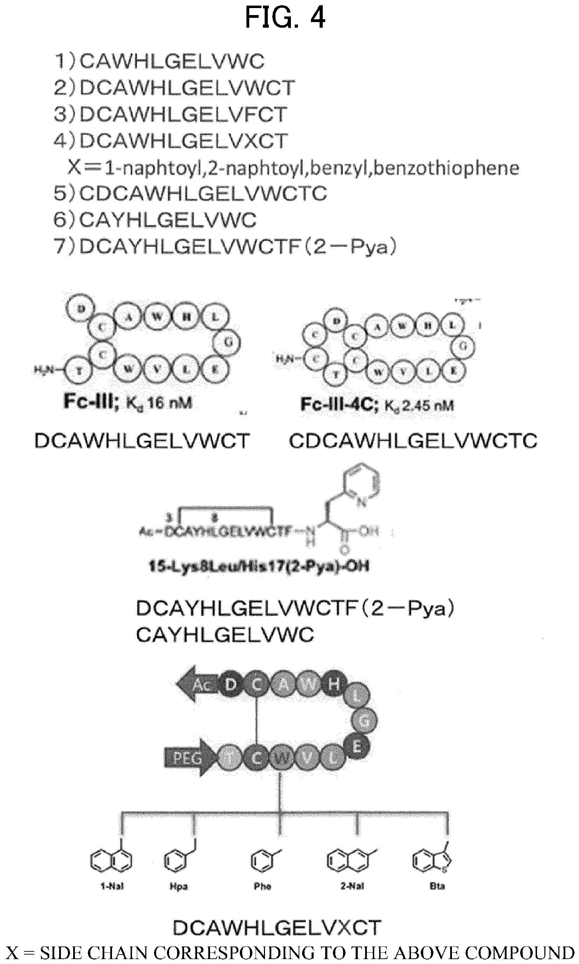

Other examples of the Fc-binding peptide can include the following peptides (FIG. 4).
1) CAWHLGELVWC (SEQ ID NO: 70)
2) DCAWHLGELVWCT (SEQ ID NO: 71)
3) DCAWHLGELVFCT (SEQ ID NO: 72)
4) DCAWHLGELVXCT (SEQ ID NO: 73)
   X = 1-naphtoyl, 2-naphtoyl, benzyl, or benzothiophene
5) CDCA WHLGEL VWCTC (SEQ ID NO: 74)
6) CAYHLGELVWC (SEQ ID NO: 75)
7) DCAYHLGELVWCTF(2-Pya) (SEQ ID NO: 76)

For example, a reactive functional group (preferably azide group) may be, if necessary, bound to the N-terminal or C-terminal (preferably, N-terminal) of the peptide for drug-binding herein, via a linker. For example, a reactive functional group (for example, azide group) is contained at the terminal of a peptide where 1 to 3 (preferably, 2) glutamic acids are further bound at the N-terminal and/or C-terminal. A peptide having an azide group can be subjected to click reaction with other functional molecule having Dibenzylcyclooctyne (DBCO), alkyne, and TCO, to result in linking of such other functional molecule to the peptide. The binding of the peptide and such other functional substance can also be performed by other method known by those skilled in the art, for example, reaction of a maleimide group and a sulfhydryl group.

Other functional molecule may also be bound to the peptide herein. For example, such other molecule can be bound via the reactive functional group (for example, to the amino terminal or the like), can be bound to the reactive functional group (for example, azide group as a substituent in a lysine residue) in the case where an amino acid (for example, lysine residue) in the peptide has the reactive functional group, or can be bound to a Cys residue (for example, Cys residue in Linker 2) in the peptide, via a maleimide group.

Other functional molecules capable of being bound to the peptide herein include a label substance or a medical drug each containing a peptide, protein, nucleic acid, or low-molecular pharmaceutical, but are not limited thereto. Any molecule where antigen specificity and other properties of an Fc molecule can be applied can be bound as such other molecule. Examples of such a substance include an anticancer agent, a low-molecule pharmaceutical product, a radiation label, a fluorescent label, a nucleic acid pharmaceutical, a gene therapy drug, a peptide pharmaceutical, and an antibody such as IgA or VHH.

The peptide for carrier-binding has at least one amino group (-NH²) for a covalent bond with an antibody. Such an amino group is preferably an amino group at the amino terminal, and may be any side-chain amino group of a lysine residue, a cysteine residue, an aspartic acid residue, a glutamic acid residue, 2-aminosuberic acid, diaminopropionic acid, and an arginine residue.

The crosslinking by the crosslinking agent is strong in resistance to alkali, and thus the crosslinking method according to the present embodiment can be a method involving improving alkali resistance of a protein or peptide or its fused product having two or more SH groups. The crosslinking method according to the present embodiment may be, for example, a method for improving alkali resistance of a disulfide bond, a method for improving alkali resistance of a protein or peptide having a disulfide bond in its molecule, or a method for enhancing stability. Specifically, a method for improving alkali resistance of a protein or peptide, including binding two thiol groups in the protein or peptide by the method, is provided.

### (IgG-Fc region-containing molecule where crosslinked Fc-binding peptide is bound)

The Fc-binding peptide can be bound with an IgG-Fc region-containing molecule. Accordingly, a complex according to another embodiment is a complex of the Fc-binding peptide intramolecularly crosslinked by the crosslinking agent and the IgG-Fc region-containing molecule. The complex encompasses a complex where the Fc-binding peptide crosslinked by the crosslinking agent containing the drug is bound to the IgG-Fc region-containing molecule; and a complex where the Fc-binding peptide crosslinked by the crosslinking agent containing the reactive functional group is bound to the IgG-Fc region-containing molecule.

The crosslinking agent according to the present embodiment may provide crosslinking between the Fc-binding peptide and the IgG-Fc region-containing molecule. Specifically, the Fc-binding peptide may be bound with an SH group of a cysteine residue contained in the antibody or the like, via the crosslinking agent. The Fc-binding peptide in such an antibody or the like-Fc-binding peptide crosslinked product may be further intramolecularly crosslinked, and, for example, the reactive functional group/drug may be bound to the Fc-binding peptide by intramolecular crosslinking and the Fc-binding peptide may be further crosslinked with a molecule containing an Fc region of the antibody or the like. Accordingly, a complex according to another embodiment is a complex of the Fc-binding peptide and the IgG-Fc region-containing molecule, crosslinked by the crosslinking agent. The complex encompasses a complex where the IgG-Fc region-containing molecule and the Fc-binding peptide are crosslinked by the crosslinking agent containing the drug; and a complex where the IgG-Fc region-containing molecule and the Fc-binding peptide are crosslinked by the crosslinking agent containing the reactive functional group.

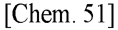

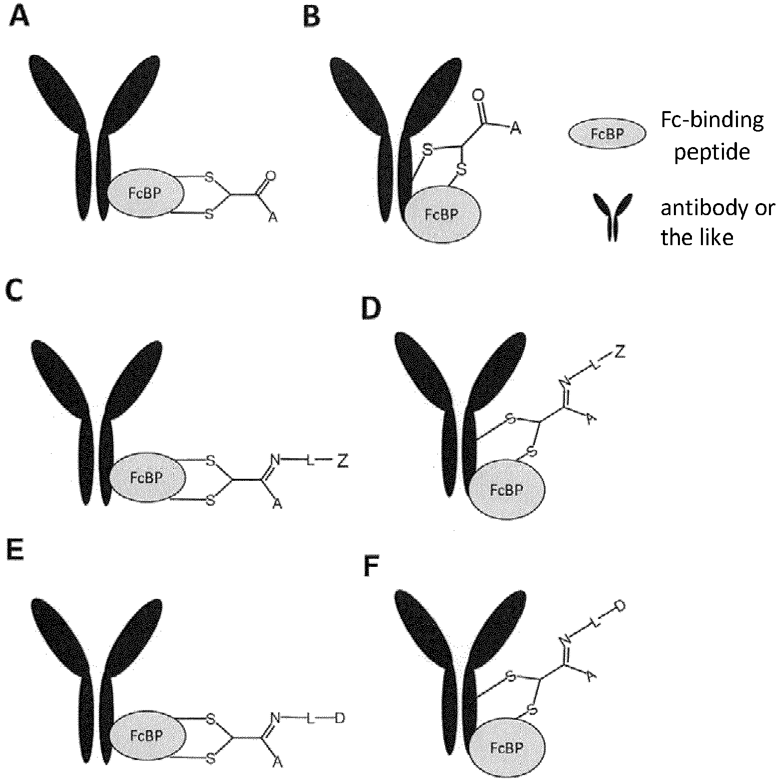

Herein, the "IgG-Fc region-containing molecule" means a peptide, a protein, or other complex, containing an Fc region of IgG, and encompasses, in addition to wild-type or artificial IgG and a mutant thereof, a fused product of an Fc region of IgG and other substance (active component, drug, protein, low-molecular compound, medium-molecular compound, high-molecular compound, matrix, lipid, liposome, nanoparticle, vehicle for DDS, nucleic acid and/or peptide), typified by an Fc fusion protein, and a molecule composed of only an Fc region. For example, when an Fc molecule is an Fc fusion protein, examples of the protein or peptide to be fused with Fc include a receptor, cytokine, interleukin, factor VIII, CTLA4, human lactoferrin, a TNF receptor, or LFA-3, or a portion thereof (preferably target-bound portion).

The complex can be produced by contacting the Fc-binding peptide crosslinked by the crosslinking agent, with the IgG-Fc region-containing molecule. Accordingly, the method for producing the IgG-Fc region-containing molecule where the drug is fused includes contacting the IgG-Fc region-containing molecule with the Fc-binding peptide crosslinked by the crosslinking agent. Alternatively, the complex can be produced by binding the Fc-binding peptide to the IgG-Fc region-containing molecule and then performing intramolecular and/or intermolecular crosslinking by the crosslinking agent.

Accordingly, the method for producing the IgG-Fc region-containing molecule is a method for producing an IgG-Fc region-containing molecule to which a crosslinked Fc-binding peptide is bound, comprising:
reacting an Fc-binding peptide bound to an IgG-Fc region-containing molecule with the crosslinking agent, to bind two thiol groups in the Fc-binding peptide via the following group: 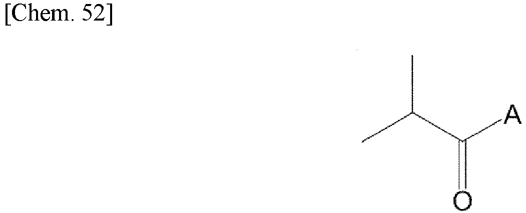
wherein A is a hydrogen atom, a C1 to 6 alkyl group optionally substituted with a phenyl group or a halogen atom, a phenyl group, or a C1 to 6 alkyl group.

The method for producing the IgG-Fc region-containing molecule is a method for producing an IgG-Fc region-containing molecule where an Fc-binding peptide where a reactive functional group Z is bound, the Fc-binding peptide being represented by the following formula: 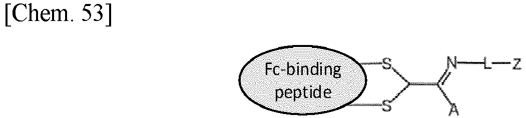
wherein A is a hydrogen atom, a C1 to 6 alkyl group optionally substituted with a phenyl group or a halogen atom, a phenyl group, or a C1 to 6 alkyl group, L represents a linker and Z represents a reactive functional group; is bound,
the method including:
   reacting an IgG-Fc region-containing molecule where a crosslinked Fc-binding peptide represented by the following formula is bound: 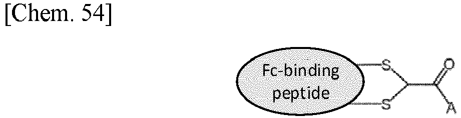
wherein A is a hydrogen atom, a C1 to 6 alkyl group optionally substituted with a phenyl group or a halogen atom, a phenyl group, or a C1 to 6 alkyl group;
and NH₂-L-Z (L represents a linker and Z represents a reactive functional group), to thereby introduce the reactive functional group into a crosslinked portion in the crosslinked Fc-binding peptide.

The method for binding the Fc-binding peptide with the antibody or the like can be performed with reference to International Publication No. WO 2013/027796, International Publication No. WO 2018/092867, International Publication No. WO 2020/075670, and/or the like.

An amino acid in a lysine residue, a cysteine residue, an aspartic acid residue, a glutamic acid residue, 2-aminosuberic acid, diaminopropionic acid, an arginine residue (preferably, lysine residue) or an amino acid at the 1-position in the Fc-binding peptide may be optionally modified with a moiety for covalent binding with the antibody, and may be covalently bound with the antibody or the like on the moiety when the peptide is bound with the antibody or the like. Herein, the Fc-binding peptide modified with the moiety is sometimes referred to as "CCAP reagent". Herein, the "moiety for covalent binding with the antibody" refers to a chemical structure for linking the Fc-binding peptide and the IgG-Fc region-containing molecule by a covalent bond, and can be a chemical structure having at least one site capable of binding with a desired amino acid (for example, a lysine residue, a cysteine residue, an aspartic acid residue, a glutamic acid residue, 2-aminosuberic acid, diaminopropionic acid, or an arginine residue). Examples of the compound imparting the moiety for covalent binding with the antibody can include DSG (disuccinimidyl glutarate), DSS (disuccinimidyl suberate), DMA (dimethyl adipimidate dihydrochloride), DMP (dimethyl pimelimidate dihydrochloride), DMS (dimethyl suberimidate dihydrochloride), DTBP (dimethyl 3,3'-dithiobispropionimidate dihydrochloride), and DSP (dithiobis(succinimidyl propionic acid)), and DSG, DSS, or DSP is preferable. For example, a succinimidyl group such as DSS or DSG is reactive with a primary amine present in a side chain of a lysine residue and the N-terminal of a polypeptide, and thus only a side chain of a lysine residue of IgBP can be specifically modified with DSS or DSG by blocking the N-terminal of the Fc-binding peptide and then performing reaction with DSS or DSG. The crosslinking between the Fc-binding peptide and IgG can site-specifically occur, for example, between an amino acid residue of X⁵, X⁹, X¹¹, X¹², or X¹⁴ of the Fc-binding peptide, and Lys248 or Lys246 in an Fc region of IgG, preferably Lys248.

The binding between the Fc-binding peptide as a CCAP reagent and the antibody or the like is not particularly limited as long as the binding is performed under conditions allowing for the occurrence of crosslinking reaction, and can be made by, for example, reaction of the Fc-binding peptide and the antibody or the like by mixing in an appropriate buffer at room temperature (for example, about 15°C to 30°C). The mixing step may be performed by, if necessary, addition of a proper amount of a catalyst promoting the crosslinking reaction. The mixing ratio between the Fc-binding peptide and the antibody or the like in the mixing step can be 1:1 to 20:1, preferably 2:1 to 20:1 or 5:1 to 10:1, for example, in terms of the molar ratio of Fc-binding peptide: antibody or the like. The mixing time (reaction time) in the mixing step can be, for example, 1 minute to 5 hours, preferably 10 minutes to 2 hours, or 15 minutes to 1 hour. The resulting bound product can be, if necessary, further purified.

An Fc region of IgG or the like usually forms a heavy-chain constant region that is a symmetric pair of two, and thus two sites where the Fc-binding peptide is to be bound can be present. Accordingly, one to two peptides, preferably one peptide of the Fc-binding peptide can be bound to one molecule of the IgG-Fc region-containing molecule.

### (Crosslinked Fc-binding peptide bond carrier)

The Fc-binding peptide can be bound to a carrier of a column or the like and thus used for purification of the antibody or the like. The crosslinking method in the present disclosure results in an enhancement in resistance to alkali, and thus the Fc-binding peptide crosslinked by the crosslinking agent of the present disclosure can be used to thereby reuse a carrier by washing with alkali once or several times. Accordingly, one aspect provides a carrier where the Fc-binding peptide intramolecularly crosslinked by the crosslinking agent is bound. The binding of the peptide to the carrier can be performed by, for example, reaction of the peptide with a carrier having a functional group reactive with an amino group. The reaction is performed under conditions imparting sufficient binding of both, and can be performed by, for example, contacting them in a buffer at room temperature for 1 to 5 hours (preferably, 2.5 to 3.5 hours).

Examples of the carrier include forms of gel (for example, gel for columns), particles, beads, nanoparticles, fine particles, macrobeads, membrane, microplate, and array, and examples of the material thereof include a magnetic substance, latex, agarose, glass, cellulose, sepharose, nitrocellulose, polystyrene, and other polymer material. The carrier is preferably a gel for columns (column chromatography). The carrier here used can be, for example, HiTrap NHS-activated HP (GE Healthcare).

A method for purifying the IgG-Fc region-containing molecule by use of the carrier is also provided. A method for purifying the antibody or the like in one aspect includes contacting an antibody or the like-containing liquid with the carrier to thereby bind the antibody or the like to the carrier, washing and removing a component not bound to the carrier, and eluting and recovering a component bound to the carrier.

The contacting between the antibody or the like-containing liquid and the carrier is performed under conditions so that both can be sufficiently contacted. For example, when the carrier is a column, the contacting is performed by injecting the antibody or the like-containing liquid into the column. The removal of the component not bound to the carrier can be performed by an ordinary method, and can be performed by, for example, washing the carrier bound to the antibody or the like, with a buffer (pH about 7.0). The recovery of the antibody or the like bound to the carrier can be performed at a pH of 2.5 or more, and the degree of acidity is desirably weak in order to prevent the antibody or the like from being denatured and the pH is preferably 3.6 or more and can be, for example, 3.6 to 4.3. Alternatively, when beads are used in the carrier, the antibody or the like can be recovered by contacting the antibody or the like and the carrier, then recovering the beads by centrifugation or the like and re-suspending them in an eluate.

### (Medical composition)

Another aspect provides a medical composition containing a peptide and/or a protein crosslinked by the crosslinking agent or an IgG-Fc region-containing molecule where an Fc-binding peptide crosslinked by the crosslinking agent of the present disclosure is bound, as an active ingredient, in particular, a therapeutic drug, a prophylactic drug, or a diagnostic product. A peptide or protein crosslinked by a crosslinking agent where a drug having a therapeutic or prophylactic effect is bound can be used in a medical (therapeutic or prophylactic) composition.

When the above peptide and/or protein, or drug D bound via the crosslinking agent are/is each a therapeutic drug, a prophylactic drug, or a drug serving as a vaccine, the peptide and/or protein crosslinked, or the antibody or the like where the peptide and/or protein crosslinked are/is bound can be used for therapy or prophylaxis.

When the above peptide and/or protein, or drug D bound via the crosslinking agent are/is each a drug serving as a label, the peptide and/or protein crosslinked, or the antibody or the like where the peptide and/or protein crosslinked are/is bound can be used for diagnosis or detection.

When the above composition is a therapeutic or prophylactic composition, the drug is a therapeutic agent or prophylactic agent, and when the above composition is a diagnostic product, the drug is a label substance. The target disease of the medical composition can be appropriately set by selecting the peptide, the protein, or the antibody or the like to be used, and the drug to be bound, and examples thereof include cancer, inflammatory disease, infection, and neurodegenerative disease.

For example, the medical composition can be utilized as an injection preparation, and encompasses dosage forms such as an intravenous injection preparation, a subcutaneous injection preparation, an intradermal injection preparation, an intramuscular injection preparation, and a drip injection preparation. Such an injection preparation can be prepared by, for example, dissolving, suspending or emulsifying an active ingredient in a sterile aqueous or oily liquid commonly used in injection preparations, according to a known method. An injection solution prepared is usually packed in an appropriate ampule, vial or syringe. The injection solution can also be obtained by adding an appropriate excipient to an active ingredient to thereby prepare a freeze-dried formulation and dissolving the formulation in an injection solvent, normal saline or the like before using. While oral administration of a protein such as an antibody is generally difficult due to decomposition by the digestive system, such oral administration can also be made by originality and ingenuity of an antibody fragment and an antibody fragment modified, and a dosage form. Examples of an oral administration formulation can include a capsule, a tablet, a syrup and a granule.

The medical composition is suitably prepared into a dosage form of a dose unit adapted to the amount of an active component administered. The dosage form of the dose unit is, for example, an injection preparation (ampule, vial, or prefilled syringe), and may usually contain 5 to 500 mg, 5 to 100 mg, 10 to 250 mg of an active ingredient or a drug per dosage form of the dose unit.

The administration route of the medical composition may be local or systemic. The administration method is not particularly limited, and is parenteral or oral administration as described above. Examples of the parenteral administration route include subcutaneous administration, intraperitoneal administration, administration into the blood (intravenous or intraarterial), or injection or dripping into the spinal fluid, and administration into the blood is preferable. The medical or diagnostic composition may be temporarily administered, or may be continuously or intermittently administered. For example, the administration can also be continuous administration for 1 minute to 2 weeks. The dosage regimen of the medical composition is not particularly limited as long as the dose and the time of administration are set so as to impart a desired therapeutic effect or prophylactic effect, and can be appropriately determined depending on the symptom, the sex, the age, and the like. For example, a single dose of the active ingredient is advantageously administered approximately once to ten times a day, preferably once to five times a day as usually about 0.01 to 20 mg/kg body weight, preferably about 0.1 to 10 mg/kg body weight, further preferably about 0.1 to 5 mg/kg body weight of an intravenous injection, before and/or after the development of clinical symptoms of the disease. Also in cases of other parenteral administration and oral administration, the administration can be made in a similar dose thereto.

Hereinafter, the present disclosure is more specifically described with reference to Examples, but the present disclosure is not limited by these Examples. The entire of the Documents cited throughout the present specification is herein incorporated by reference.

### (Example 1) Preparation of 1,1-dichloroacetone-crosslinked cyclic peptide

A raw material peptide [sequence: Fmoc-HN-GSGGS-GPDCA YHRGEL VWCTFH (SEQ ID NO: 1): IgGBP-longGS or IgGBP-LGS] was synthesized on commission (Eurofins) by a solid-phase peptide synthesis method (Fmoc method). In 1500 µL of DMF was dissolved 5 mg (1.95 µmol) of the raw material peptide, TCEP-HCl (1.12 mg, 3.9 µmol, 2 equivalent moles) dissolved in 2 ml of PBS (pH 7.4) in advance was added thereto, and reduction reaction was performed under stirring at room temperature for 30 minutes. Thereafter, 1,1-Dichloro-2-propanone (0.495 mg, 3.9 µmol, 2 equivalent moles) dissolved in 120 µL of acetonitrile was added, and the resultant was stirred at room temperature. After 1 hour, the termination of the reaction was confirmed by LC-MS analysis (LC-MS8030 manufactured by SHIMADZU CORPORATION), and the reaction solution was purified by HPLC (C18 reverse-phase column), to thereby obtain an Fmoc-cyclized peptide (2 mg, 0.76 µmol, 40% yield). Thereafter, 2% piperidine was added for deprotection of an Fmoc protection group, the termination of the reaction was confirmed after 10 minutes by LC-MS analysis (LC-MS8030 manufactured by SHIMADZU CORPORATION), and the reaction solution was directly purified by HPLC (C18 reverse-phase column), to thereby obtain a cyclized peptide (1 mg, 0.38 µmol, 20% yield). The peptide finally purified was subjected to confirmation of the molecular weight by LC-MS analysis (LC-MS8030 manufactured by SHIMADZU CORPORATION), and then freeze-dried.

### (Example 2) Preparation of 1,1-dichloropinacolin-crosslinked cyclic peptide

A raw material peptide [sequence: Fmoc-HN-GSGGSGPDCAYHRGELVWCTFH (SEQ ID NO: 1)] was synthesized on commission (Eurofins) by a solid-phase peptide synthesis method (Fmoc method). In 1500 µL of DMF was dissolved 10 mg (3.9 µmol) of the raw material peptide, TCEP-HCl (2.24 mg, 7.8 µmol, 2 equivalent moles) dissolved in 2 ml of PBS (pH 7.4) in advance was added thereto, and reduction reaction was performed under stirring at room temperature for 30 minutes. Thereafter, 1,1-dichloropinacolin (1.32 mg, 7.8 µmol, 2 equivalent moles) dissolved in 120 µL of acetonitrile was added, and the resultant was stirred at room temperature. After 1 hour, the termination of the reaction was confirmed by LC-MS analysis (LC-MS8030 manufactured by SHIMADZU CORPORATION), and the reaction solution was purified by HPLC (C18 reverse-phase column), to thereby obtain an Fmoc-cyclized peptide (7 mg, 2.7 µmol). Thereafter, 2% piperidine was added for deprotection of an Fmoc protection group, the termination of the reaction was confirmed after 10 minutes by LC-MS analysis (LC-MS8030 manufactured by SHIMADZU CORPORATION), and the reaction solution was directly purified by HPLC (C18 reverse-phase column), to thereby obtain a cyclized peptide (5 mg, 2.04 µmol, 50% yield). The peptide finally purified was subjected to confirmation of the molecular weight by LC-MS analysis (LC-MS8030 manufactured by SHIMADZU CORPORATION), and then freeze-dried.

### (Example 3) Preparation of 2,2-dichloroacetophenone-crosslinked cyclic peptide

A raw material peptide [sequence: Fmoc-HN-GSGGS-GPDCA YHRGEL VWCTFH (SEQ ID NO: 1)] was synthesized on commission (Eurofins) by a solid-phase peptide synthesis method (Fmoc method). In 1500 µL of DMF was dissolved 2.5 mg (1.00 µmol) of the raw material peptide, TCEP-HCl (0.506 mg, 2.0 µmol, 2 equivalent moles) dissolved in 2 ml of PBS (pH 7.4) in advance was added thereto, and reduction reaction was performed under stirring at room temperature for 30 minutes. Thereafter, 2,2-dichloroacetophenone (0.38 mg, 2.0 µmol, 2 equivalent moles) dissolved in 120 µL, of acetonitrile was added, and the resultant was stirred at room temperature. After 1 hour, the termination of the reaction was confirmed by LC-MS analysis (LC-MS8030 manufactured by SHIMADZU CORPORATION), and the reaction solution was purified by HPLC (C18 reverse-phase column), to thereby obtain about 2 mg of an Fmoc-cyclized peptide. Thereafter, 2% piperidine was added for deprotection of an Fmoc protection group, the termination of the reaction was confirmed after 10 minutes by LC-MS analysis (LC-MS8030 manufactured by SHIMADZU CORPORATION), and the reaction solution was directly purified by HPLC (C18 reverse-phase column), to thereby obtain a cyclized peptide (1.2 mg, 0.49 µmol, 49% yield). The peptide finally purified was subjected to confirmation of the molecular weight by LC-MS analysis (LC-MS8030 manufactured by SHIMADZU CORPORATION), and then freeze-dried.

### (Example 4) Measurement of binding affinity of crosslinked cyclic peptide

The affinity analysis was performed by the following method. First, a CM5 sensor chip was installed on BIAcoreT200 (GE healthcare), and 0.4 M 1-ethyl-3-(3-dimethylaminopropyl)carbodiimide (EDC) and a 0.1 M sulfo-N-hydroxysuccinimide (sulfo-NHS) solution mixed in equivalent amounts were injected to the sensor chip at a flow rate of 10 µl/ml, to thereby activate the sensor chip. Thereafter, IgG was immobilized onto the sensor chip under a condition of a pH of 5.5 (10 mM Na acetate). An HBS-EP buffer (10 mM HEPES, 150 mM NaCl, 0.005% Tween 20, 3 mM EDTA, pH 7.4) was used in measurement, and 15.625, 31.2, 62.5, 125, 250, and 500 nM of peptides were each injected at a flow rate of 50 µl/ml for 180 seconds, to thereby monitor binding reaction. When dissociation reaction was measured, only the buffer was injected for 600 seconds. The interaction parameter was analyzed with BIAevalution T100 software.

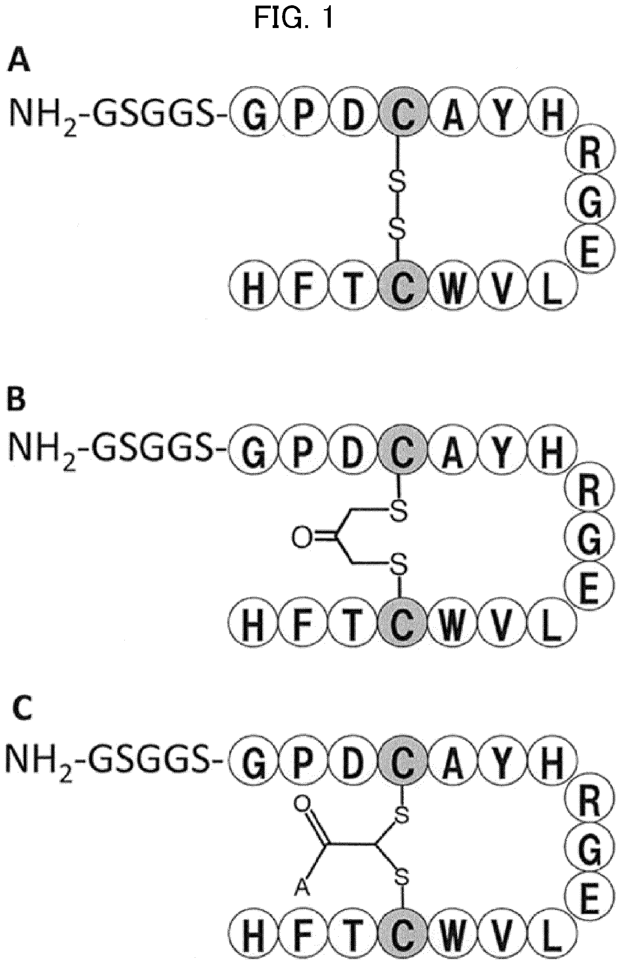

IgG-binding peptide derivatives compared and evaluated are illustrated in FIG. 1. The results of evaluation of the affinities of these peptides with human IgG1 are shown in Table 1. The Kd value of the affinity of the original IgG-binding peptide having a disulfide bond was 8.2 nM, and that of a 1,3-dichloroacetone-crosslinked cyclic peptide was 4.9 µM and the affinity was reduced by about 500-fold. In this regard, the Kd value of a 1,1-dichloroacetone-crosslinked cyclic peptide was 45.6 nM and was reduced by about 5-fold as compared with that of the original peptide, the Kd value of a 1,1-dichloropinacolin-crosslinked cyclic peptide was 112 nM and was reduced by about 14-fold as compared with that of the original peptide, and the Kd value of a 2,2-dichloroacetophenone-crosslinked cyclic peptide was 6.4 nM and was comparable with that of the original peptide.

**[Table 1]**

| Peptide name | Peptide sequence | Crosslinking agent name | SPR | | |
|---|---|---|---|---|---|
| | | | ka | kd | Kd value(nM) |
| IgG-BP-LongGS | GSGGSGPDCAYHRGELVWCTFH | Original | 2.37E+06 | 0.019 | 8.0 |
| | | 1,1-dichloroacetone | 1.84E+06 | 0.0604 | 45.6 |
| | | 1,1-dichloroacetophenone | 5.64E+06 | 0.03625 | 6.4 |
| | | 1,1-dichloopinacolin | 3.33E+06 | 0.0677 | 112.0 |
| | | 1,3-dichloroacetone | 100E+03 | 0.005 | 4900 |

### (Example 5) Preparation of peptide-immobilized column and purification of IgG

Five mL of 1 mM hydrochloric acid was sent to an NHS-activated prepacked column having a volume of 1 mL, and an isopropanol solution in the column was removed. Next, a 10.0 mg/mL peptide solution (dissolved in 100 µL of DMSO) was diluted with a coupling solution (20 mM carbonate buffer, 50 mM sodium chloride, pH 8.3) by 10-fold, 1 mL of the dilution liquid was sent, and immobilization was performed at room temperature for 4 hours. Thereafter, unreacted NHS was blocked by 5 mL of 1 M Tris (pH 8.0) at room temperature for 1 hour. Thereafter, washing with 5 mL of an aqueous 0.1 N NaOH solution was made. Finally, 10 mL of a PBS solution (20 mM phosphate buffer, 150 mM sodium chloride, pH 7.4) was sent, and used in chromatography evaluation.

The IgG-binding peptide-immobilized column produced was connected to a BioLogic LP (manufactured by Bio-Rad Laboratories Inc.) liquid chromatographic system, and equilibrated with PBS. Next, 1 mg/mL human serum-derived IgG (manufactured by Sigma-Aldrich Co. LLC) dissolved in PBS was sent at a flow rate of 1 mL/min for 1 minute. Furthermore, the column was washed with PBS, and an elution solution (100 mM glycine buffer, pH 2.8) was sent, to thereby elute IgG as an adsorption component. The elution of IgG from the column was detected by the absorbance at 280 nm.

### (Example 6) Dynamic binding capacity (DBC) measurement and alkali resistance evaluation 1 of peptide-immobilized column

A column where the amount of immobilization of a peptide was 1 mg was produced by the same method as described above. The column produced was equilibrated with PBS, and 1 mg/mL human serum-derived IgG (manufactured by Sigma-Aldrich Co. LLC) dissolved in PBS was sent at a flow rate of 1 mL/min (retention time 1 min). DBC was indicated as one determined from the amount of a protein added at the time of point where 10% of the absorbance at 280 nm of the sample added was leaked.

Next, 5 mL of an aqueous 0.1 N sodium hydroxide solution was sent to the 1-mL column where 1 mg of the peptide was immobilized. Thereafter, washing with PBS was performed. This cycle was repeated 30 times for the 1,1-dichloroacetone-crosslinked cyclic peptide, and was repeated 10 times for the 1,1-dichloropinacolin-crosslinked cyclic peptide. DBC measurement was performed at a flow rate of 1 mL/min at the 1st to 5th and 10th times of the cycles, and further performed at the 20th and 30th times of the cycles with respect to the 1,1-dichloroacetone-crosslinked cyclic peptide, and thus alkali resistance was evaluated. The variation in DBC was determined based on the measurement results under the assumption that DBC immediately after column production was 100% according to Table 2.

**[Table 2]**

| Number of washing times with 0.1 N NaOH | **IgG-BP-LGS/2,2-dichloroacetone (IgGBPLGS-DCA)** | | | |
|---|---|---|---|---|
| | Maximum binding capacity (mg) | IgGBPLGS-DCA Variation (%) in maximum binding capacity | Dynamic binding capacity (mg/ml) | IgGBPLGS-DCA Variation (%) in dynamic binding capacity |
| **1** | **9.68** | **100.0** | **12.78** | **100.0** |
| **2** | **9.70** | **100.2** | **12.78** | **100.0** |
| **3** | **9.88** | **102.1** | **12.18** | **95.3** |
| **4** | **10.12** | **104.5** | **12.18** | **95.3** |
| **5** | **9.93** | **102.6** | **12.38** | **96.9** |
| **10** | **9.32** | **96.3** | **12.18** | **95.3** |
| **20** | **8.35** | **86.3** | **12.38** | **96.9** |
| **30** | **8.78** | **90.7** | **11.58** | **90.6** |
| | | | | |

| Number of washing times with 0.1 N NaOH | **IgG-BP-LGS/1,1-dichloropinacolin (IgGBPLGS-DCP)** | | | |
|---|---|---|---|---|
| | Maximum binding capacity (mg) | IgGBPLGS-DCP Variation (%) in maximum binding capacity | Dynamic binding capacity (mg/ml) | IgGBPLGS-DCP Variation (%) in dynamic binding capacity |
| **1** | **12.11** | **100.0** | **16.70** | **100.0** |
| **2** | **12.09** | **99.8** | **17.20** | **103.0** |
| **3** | | | | |
| **4** | | | | |
| **5** | **10.87** | **89.8** | **16.24** | **97.2** |
| **10** | **10.55** | **87.1** | **14.46** | **86.6** |
| **20** | | | | |
| **30** | | | | |
| | | | | |

| Number of washing times with 0.1 N NaOH | **IgG-BP-LGS** | | | |
|---|---|---|---|---|
| | Maximum binding capacity (mg) | IgGBPLGS Variation (%) in maximum binding capacity | Dynamic binding capacity (mg/ml) | IgGBPLGS Variation (%) in dynamic binding capacity |
| **1** | **12.37** | **100.0** | **17.24** | **100.0** |
| **2** | | | | |
| **3** | | | | |
| **4** | | | | |
| **5** | **6.09** | **49.2** | **8.74** | **50.7** |
| **10** | | | | |
| **20** | | | | |
| **30** | | | | |

In this regard, while the initial value of DBC of each peptide column was 17.24 mg/mL-column in the case of the original peptide and was 2.3 mg/mL-column in the case of the 1,3-dichloroacetone-crosslinked peptide, the value of DBC was 12.8 mg/mL-column in the case of the 1,1-dichloroacetone-crosslinked peptide and was 16.70 mg/mL-column in the case of the 1,1-dichloropinacolin-crosslinked peptide. Accordingly, it was revealed that IgG adsorption performance was high as compared with a conventional crosslinked cyclic peptide.

### (Example 7) Dynamic binding capacity (DBC) measurement and alkali resistance evaluation 2 of peptide-immobilized column

Five mL of a 0.1 M sodium hydroxide solution was sent to the produced 1-mL column where the amount of immobilization of a peptide was 1 mg, and thereafter washing with 5 mL of PBS was performed. These operations were defined as one cycle and treatment of washing with aqueous NaOH solution/washing with PBS was performed 1 to 30 times, and thereafter DBC measurement was performed at a flow rate of 1 mL/min at the intended number of times (1, 2, 3, 4, 5, 10, 20, 30 times).

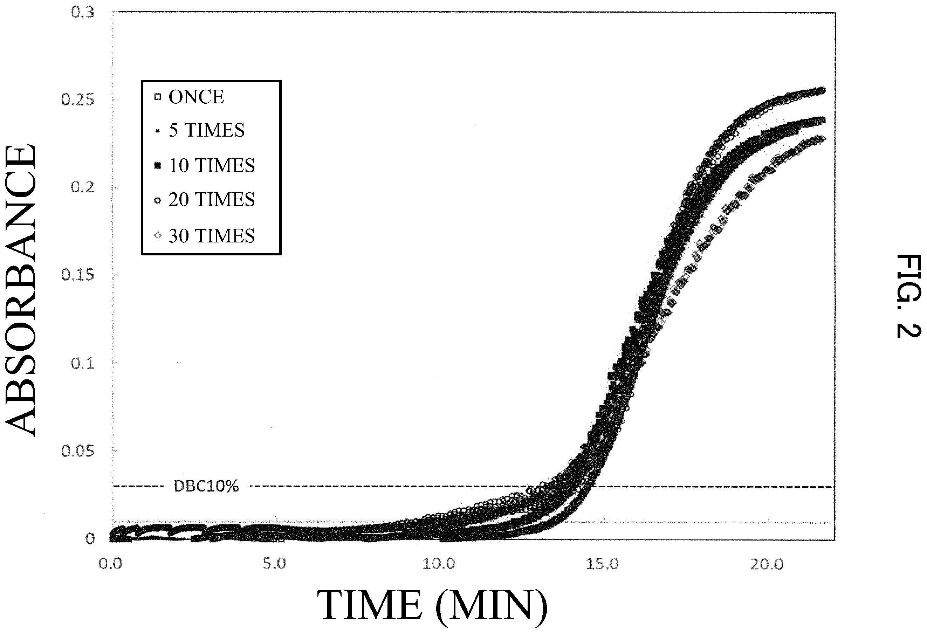

The results are illustrated in FIG. 2. It was found that, while the amount of binding of the antibody to the column was reduced by alkali washing in the case of the original IgG-binding peptide having a disulfide bond (not illustrated), a reduction in amount of binding of the antibody to the column was extremely slight in each case of the 1,1-dichloroacetone-crosslinked peptide and the 1,1-dichloropinacolin-crosslinked peptide.

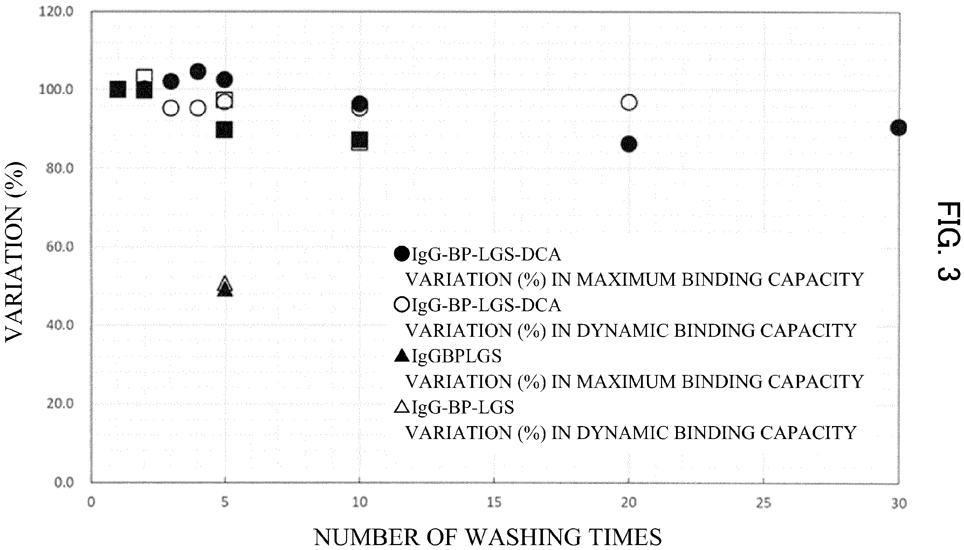

FIG. 3 illustrates the results of comparison in rate of change in amount of the antibody at a value of 10% of DBC. While the DBC of the original IgG-binding peptide-immobilized column having a disulfide bond was reduced to 50% or less in alkali washing 5 times, that of the 1,1-dichloroacetone-crosslinked peptide was kept at 90% or more in washing 30 times and that of the 1,1-dichloropinacolin-crosslinked peptide was kept at 85% or more in washing 10 times (FIG. 3). It was thus found that the 1,1-dichloroacetone-crosslinked peptide and the 1,1-dichloropinacolin-crosslinked peptide acquired clearly high alkali resistance. In this regard, while the value of DBC, by itself, of the original IgG-binding peptide-immobilized column having a disulfide bond was 17.24 mg/ml, that of the 1,1-dichloroacetone-crosslinked peptide column was 12.8 mg/ml and corresponded to about 74% and that of the 1,1-dichloropinacolin-crosslinked peptide column was 16.70 mg/ml and corresponded to about 96%. The DBC is generally varied considerably also depending on the amount of a ligand (peptide) immobilized and the flow rate, and thus columns respectively using the 1,1-dichloroacetone-crosslinked peptide and the 1,1-dichloropinacolin-crosslinked peptide are also considered to be able to be finished as columns suitable for practical use by finding optimal conditions.

### (Example 8) Introduction of reactive functional group

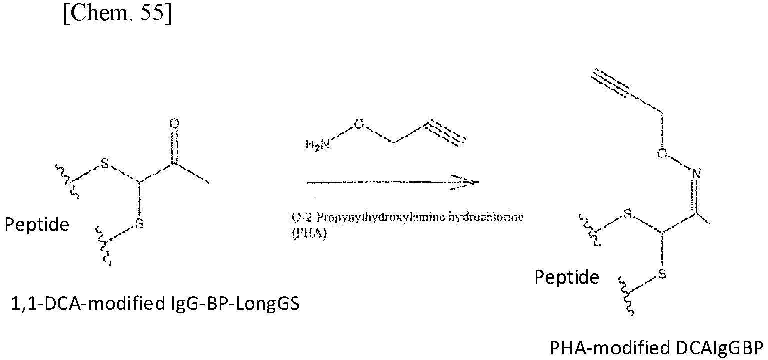

In 400 µl of DMF was dissolved 0.201 µmol of the prepared 1,1-dichloroacetone-crosslinked cyclic peptide, and a 100-fold volume of an O-2-Propynylhydroxylamine hydrochloride (20.1 µmol) solution obtained by dissolution in 1.0 ml of a 0.2 M NaHCOs buffer (pH 8.3) in advance was added thereinto. The reaction liquid was stirred at room temperature for 24 hours. The termination of the reaction was confirmed by LC-MS analysis (LC-MS8030 manufactured by SHIMADZU CORPORATION), and the reaction solution was directly purified by HPLC (C18 reverse-phase column), to thereby obtain an alkyne functional group-introduced, crosslinked and cyclized peptide (0.10 µmol, 50% yield). The peptide finally purified was subjected to confirmation of the molecular weight by LC-MS analysis (LC-MS8030 manufactured by SHIMADZU CORPORATION), and then freeze-dried. The resulting compound was subjected to the same affinity measurement as in Example 4 (Measurement of binding affinity of crosslinked cyclic peptide), and as a result, the Kd value was 334 nM and was reduced by about 42-fold as compared with that of the original peptide.

### (Example 9) Crosslinking of VHH antibody with 1,1-dichloroacetone

A urea solution was added to 100 µl (95 µg, 56 × 10⁻¹⁰ mol) of a PBS solution (pH 7.4) of a 950 µg/ml anti-CD89 VHH antibody (IgARC25) so as to have a final concentration of 5 M, and the mixture was left to still stand at room temperature for 1 hour. Thereafter, TCEPTCEP-HCl (11.2 nmol, 2 equivalent moles) was added, and reduction reaction was performed under stirring at room temperature for 30 minutes. Thereafter, 1,1-Dichloroacetone (11DCA, 11.2 nmol, 2 equivalent moles) dissolved in 120 µL, of acetonitrile was added, and the mixture was stirred at room temperature. After 1 hour, the termination of the reaction was confirmed by LC-MS analysis (Bio-Accord SYSTEM manufactured by Waters Corporation). As a result, the molecular weight of IgARC25 was 14,028 Da before crosslinking, and was 14,085 Da after treatment with 11DCA and was found to be increased by 57 Da, and crosslinking by 11DCA was thus confirmed.

### (Example 10) Production of peptide having crosslinked structure by peptide synthesis

In crosslinking reaction of a disulfide bond by a 1,1-dichloroacetone derivative compound (1,1-dichloroacetone, 1,1-dichloropinacolin, 2,2-dichloroacetophenone) represented by the following formula, 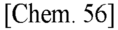 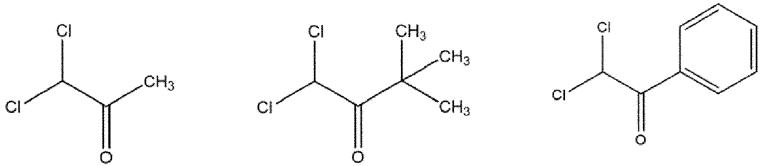 a peptide having two Cyses each modified by Fmoc at the N-terminal (or a peptide obtained by reduction of a peptide modified by Fmoc at the N-terminal and having a disulfide bond) and 1.0 to 2.0 equivalents of the 1,1-dichloroacetone derivative compound were reacted in PBS to thereby perform crosslinking (step a in scheme A), and finally Fmoc at the N-terminal was removed and deprotected (step b in scheme A) for preparation, as shown in the following formula. On the other hand, a method not according to this method was designed where crosslinking reaction was performed in peptide synthesis by am Fmoc method, as shown in scheme B. A peptide containing one of two Cyses to be crosslinked was synthesized from the C-terminal to the previous one of the other Cys, on a peptide synthesis resin by an Fmoc method. The protection group of Cys in the peptide was deprotected (step c in scheme B), and then Fmoc-chloroacetophenoyl cysteine was added and linked (step d in scheme B). Fmoc was deprotected (step e in scheme B), and the α-amino group at the N-terminal of the peptide generated, and the α-carboxyl group at the acetophenoyl cysteine side were coupled (step f in scheme B). The remaining amino acids were subjected to linking and synthesis by an Fmoc method (step g in scheme B), and finally an objective peptide was obtained by cleavage from the resin and deprotection of Fmoc.

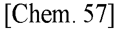

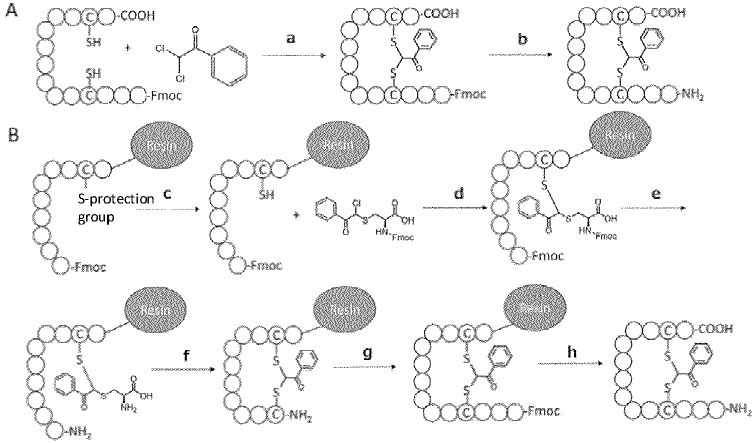

### (Synthesis of Fmoc-allylated chloroacetophenoyl cysteine)

Fmoc-chloroacetophenoyl cysteine (hereinafter, compound 5) used in the present method was synthesized by the following method. In the following, the α-carboxyl group of the Fmoc-chloroacetophenoyl cysteine was protected by an allyl group.

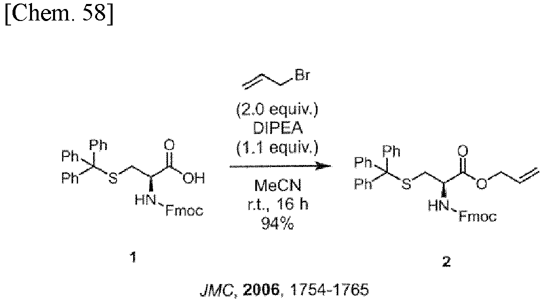

Under an argon stream, N,N-diisopropylethylamine (1.60 mL, 9.38 mmol, 1.1 equivalents) was added to an acetonitrile solution (85 mL) of a compound 1 (5.00g, 8.53 mmol) and allyl bromide (1.44 mL, 17.1 mmol, 2 equivalents), and the mixture was stirred at room temperature for 16 hours. After termination of the reaction by addition of water, the reaction liquid was concentrated and then extracted with ethyl acetate, and the organic layer was washed with an aqueous saturated sodium hydrogen carbonate solution, an aqueous 2 N hydrochloric acid solution and saturated saline, and then dried by sodium sulfate. The resultant was concentrated and the resulting residue was purified with silica gel column chromatography (hexane: ethyl acetate = 4:1), to thereby obtain a compound 2 (5.03g, 94%).

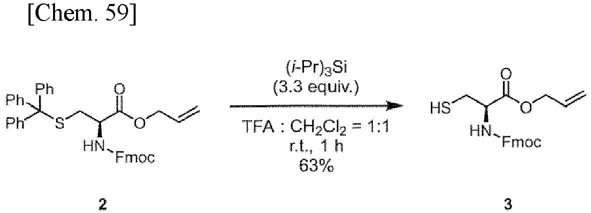

The compound 2 (5.03g, 8.03 mmol) and triisopropylsilane (5.45 mL, 26.5 mmol, 3.3 equivalents) were added to a solution of trifluoroacetic acid and dichloromethane at 1:1 (30 mL), and the mixture was stirred at room temperature for 1 hour. After completion of the reaction and then azeotropy with toluene, the residue obtained by concentration was purified with silica gel column chromatography (hexane: ethyl acetate = 4:1), to thereby obtain a compound 3 (1.94g, 63%).

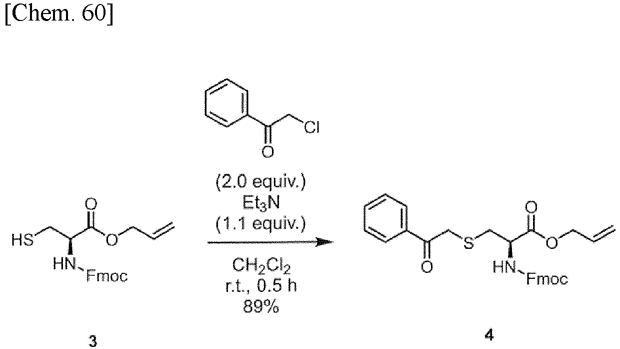

Under an argon stream, triethylamine (0.401 mL, 2.90 mmol, 1.1 equivalents) was added to a dichloromethane solution (26 mL) of the compound 3 (1.00g, 2.63 mmol) and phenacyl chloride (814 mg, 5.27 mmol, 2 equivalents), and the mixture was stirred at room temperature for 30 minutes. After termination of the reaction by addition of water, extraction with dichloromethane was made, and the organic layer was washed with saturated saline and then dried by sodium sulfate. The resultant was concentrated and the resulting residue was purified with silica gel column chromatography (hexane: ethyl acetate = 2:1), to thereby obtain a compound 4 (1.17g, 89%).
1H NMR (400 MHz, CDCl₃, ppm): δ7.96 (d, J = 7.8 Hz, 2H),7.76 (d, J = 7.3 Hz, 2H),7.64-7.57 (m, 3H),7.47 (dd, J = 7.6,8.0 Hz, 2H),7.40 (dd, J = 7.3,7.8 Hz, 2H),7.31 (ddd, J = 1.4,7.3,7.3 Hz, 2H),5.94-5.84 (m, 2H),5.33 (d, J = 16.9 Hz, 1H),5.24 (dd, J = 1.4,10.1 Hz, 1H),4.71-4.64 (m, 3H),4.40-4.38 (m, 2H),4.23 (dd, J = 6.9,6.9 Hz, 1H), 3.90 (s, 2H), 3.16-3.03 (m, 2H)
HRMS (FAB-TOF) m/z: [(M+H)⁺] calcd for C₂₉H₂₈N₁O₅S₁ 502.1688; found502.1690.

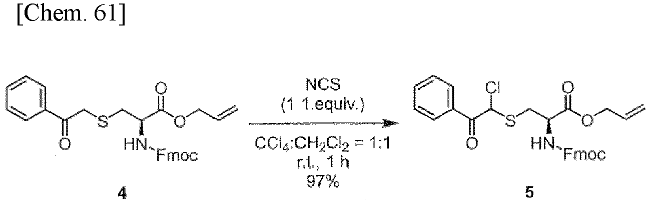

Under an argon stream, a solution of carbon tetrachloride and dichloromethane at 1:1 (4 mL), of the compound 4 (204 mg, 0.401 mmol) and N-chlorosuccinimide (59.6 mg, 0.447 mmol, 1.1 equivalents), was added, and stirred at room temperature for 1 hour. After termination of the reaction by addition of water, the aqueous layer was extracted with dichloromethane, and the organic layer was washed with saturated saline and then dried by sodium sulfate. The resultant was concentrated and the resulting residue was purified with silica gel column chromatography (only dichloromethane), to thereby obtain a compound 5 (210 mg, 97%).
1H NMR (400 MHz, CDCl₃, ppm): δ7.99 (d, J = 7.8 Hz, 2H), 7.76 (d, J = 7.3 Hz, 2H), 7.63-7.57 (m, 3H), 7.48 (dd, J = 7.8,7.8 Hz, 2H), 7.39 (dd, J = 7.3,7.8 Hz, 2H), 7.30 (ddd, J = 1.0,7.3,7.3 Hz, 2H), 6.37 (d, J = 25.6 Hz, 1H), 5.95-5.84 (m, 1H), 5.63-5.58 (m, 1H), 5.38-5.24 (m, 2H), 4.76-4.60 (m, 3H), 4.39 (d, J = 6.9 Hz, 2H), 4.21 (dd, J = 6.9,6.9 Hz, 1H), 3.51-3.14 (m, 2H)
HRMS (FAB-TOF) m/z: [(M+H)⁺] calcd for C₂₉H₂₇N₁O₅S₂Cl₁S₁ 536.1296; found 536.1298.

### (Reactivity of Fmoc-allylated chloroacetophenoyl cysteine and Cys)

Whether or not the Fmoc-allylated chloroacetophenoyl cysteine (compound 5) synthesized had reactivity with thiol of Cys was tested by the following reaction.

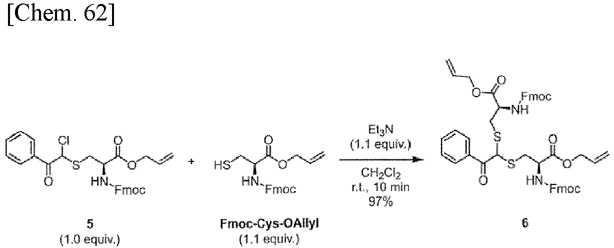

Under an argon stream, triethylamine (0.017 mL, 0.121 mmol, 1.1 equivalents) was added to a dichloromethane solution (1.1 mL) of the compound 5 (59.0 mg, 0.110 mmol) and Fmoc-Cys-OAllyl (0.046 mg, 0.121 mmol, 1.1 equivalents), and the mixture was stirred at room temperature for 10 minutes. After termination of the reaction by addition of water, the aqueous layer was extracted with dichloromethane, and the organic layer was washed with saturated saline and then dried by sodium sulfate. The resultant was concentrated and the resulting residue was purified with silica gel column chromatography (hexane: ethyl acetate = 3:1), to thereby o btain a compound 6 (93.8 mg, 97%).
1H NMR (400 MHz, CDCl₃, ppm): δ7.96 (d, J = 7.8 Hz, 2H), 7.75 (dd, J = 2.7,7.8 Hz, 4H), 7.59-7.53 (m, 5H), 7.41 (dd, J = 7.8,7.8 Hz, 2H), 7.39-7.35 (m, 4H), 7.30-7.26 (m, 4H), 5.91-5.80 (m, 2H), 5.70 (dd, J = 7.8,22.0 Hz, 2H), 5.61 (s, 1H), 5.31 (d, J = 17.4 Hz, 2H), 5.22 (d, J = 11.6 Hz, 2H), 4.69-4.59 (m, 6H), 4.41-4.29 (m, 4H), 4.19 (dd, J = 7.3,7.3 Hz, 2H), 3.33-2.97 (m, 4H),
HRMS (FAB-TOF) m/z: [(M+Na)⁺] calcd for C₅₀H₄₆N₂O₉S₂Na 905.2542; found 905.2542.

The above results indicated that the Fmoc-allylated chloroacetophenoyl cysteine (compound 5) had reactivity with thiol of Cys, and the progress of the reaction shown in step d in scheme B was demonstrated. It was verified from the above results that synthesis of a novel crosslinked peptide in scheme B could be carried out.

### (Example 11) Crosslinking of oxytocin

Crosslinking of oxytocin (Cys-Tyr-Ile-Gln-Asn-Cys-Pro-Leu-Gly-NH₂ (SEQ ID NO: 77), intramolecular SS bond, molecular weight: 1007.19) was performed with 1.1-dichloroacetone. Ten mg (9.92 µmol) of acetate (manufactured by Toronto Research Chemicals) in an oxytocin SS oxidized form (oxytocin-OX), shown by the following formula A, was dissolved in 3 mL of a 0.1 M HEPES-HCl buffer (pH 8.0), 10.2 mL of a 0.1 M HEPES-HCl buffer (pH 8.0) where 57.1 mg (200 µmol) of TCEP hydrochloride (Tris(2-carboxyethyl)phosphine Hydrochloride) as a reducing agent was dissolved was mixed, and the mixture was stirred for 1 hour. Thereto was added 2.77 mg (21.8 µmol) (molar ratio to oxytocin: 2.2) of 1,1-dichloroacetone dissolved in 0.722 mL of acetonitrile, and the mixture was stirred for 1 hour.

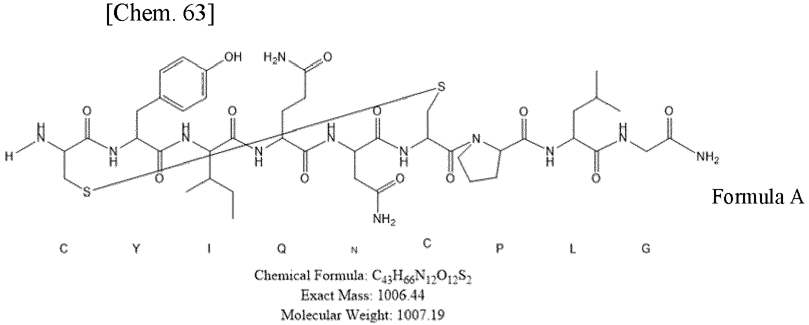

The reaction product obtained was analyzed by LC-MS as follows. The reaction product was diluted with 0.1% formic acid by 5-fold, and thereafter 20 µL thereof was subjected to analysis (flow rate: 0.2 mL/min, elution: linear gradient from 4% CH₃CN containing 0.1% formic acid, to 70% CH₃CN, column temperature: 25°C) with an Acquity UPLC/SQ detector system (manufactured by Waters Corporation) jointed with a Peptide BEH-C18 column (130Å, 1.7 µm, 2.1 × 100 mm, manufactured by Waters Corporation).

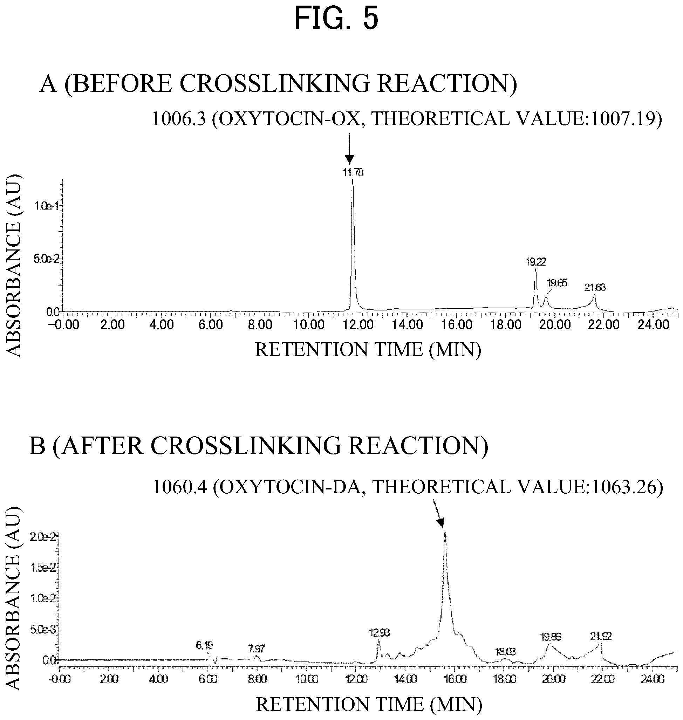

The respective results of oxytocin-OX before addition of TCEP hydrochloride, and the reaction product, analyzed with LC-MS, are illustrated in FIG. 5A and FIG. 5B. The measurement value of the mass at the peak assigned to oxytocin-OX was 1006.3, and was almost identical to 1007.19 as the theoretical value with respect to oxytocin-OX. On the other hand, the peak assigned to the reaction product in the crosslinking reaction of 1.1-dichloroacetone was eluted behind the original peak, and the mass at the peak was 1060.4. The mass was almost identical to 1063.26 as the theoretical value with respect to an oxytocin-dichloroacetone-crosslinked form (oxytocin-DA) shown in formula A1, and thus a crosslinked product having an objective structure was determined to be obtained.

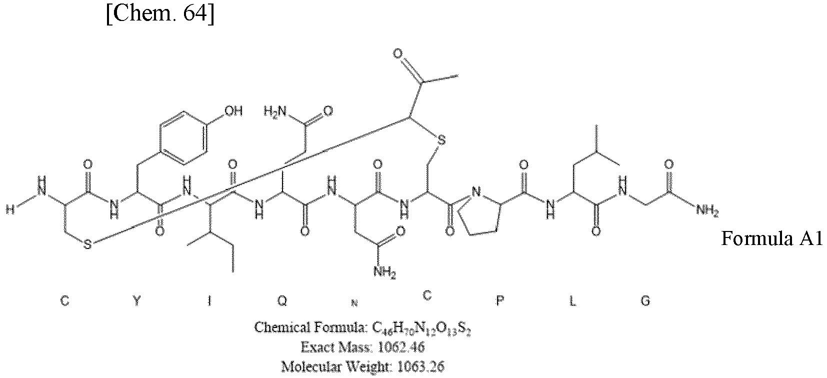

### (Example 12) Crosslinking of vasopressin

Crosslinking of vasopressin (Cys-Tyr-Phe-Gln-Asn-Cys-Pro-Arg-Gly-NH₂ (SEQ ID NO: 78), intramolecular SS bond, molecular weight: 1084.24) was performed with 1.1-dichloroacetone. In 1.65 mL of a 0.1 M phosphate buffer (pH 8.0) was dissolved 5.5 mg (5.07 µmol) of acetate (manufactured by Tokyo Chemical Industry Co., Ltd.) of a vasopressin SS oxidized form (vasopressin-OX), shown by the following formula B, 4.4 mL of a 0.1 M phosphate buffer (pH 8.0) where 24.6 mg (86.0 µmol) of TCEP hydrochloride was dissolved was mixed, and the mixture was stirred for 1 hour. Thereto was added 1.42 mg (11.2 µmol) (molar ratio to vasopressin: 2.2) of 1,1-dichloroacetone dissolved in 0.371 mL of acetonitrile, and the mixture was stirred for 1 hour.

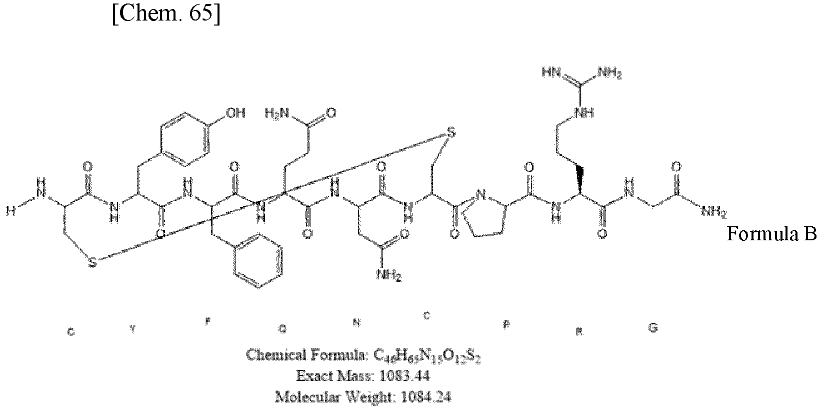

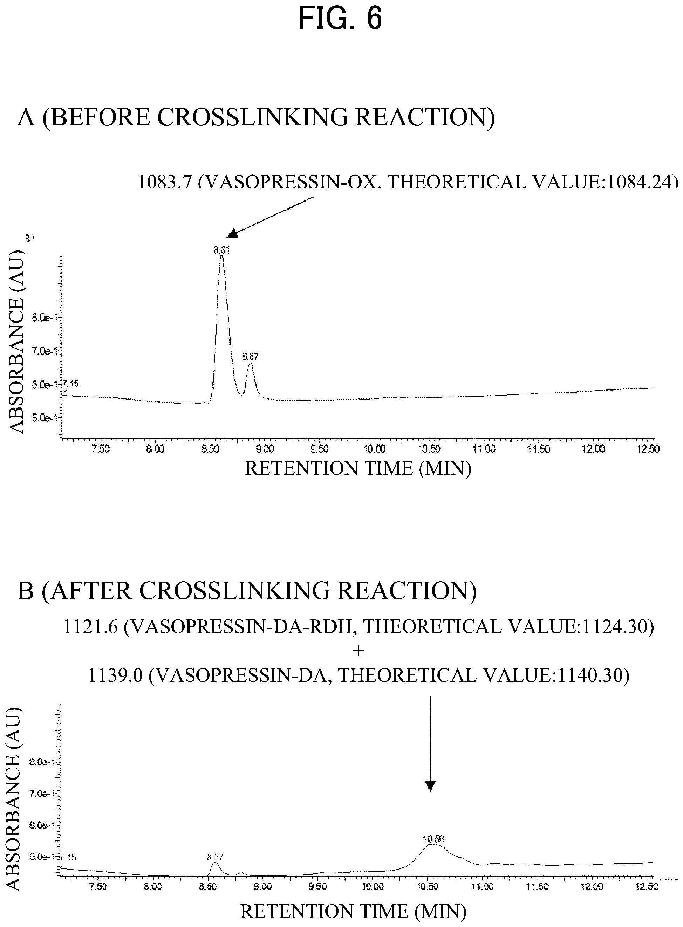

The reaction product obtained was analyzed by LC-MS in the same manner as in Test Example 11. The respective results of vasopressin-OX before addition of TCEP hydrochloride, and the reaction product, analyzed with LC-MS, are illustrated in FIG. 6A and FIG. 6B. The measurement value of the mass at the peak assigned to vasopressin-OX was 1083.7, and was almost identical to 1084.24 as the theoretical value with respect to vasopressin-OX. On the other hand, the peaks assigned to the reaction product in the crosslinking reaction of 1.1-dichloroacetone were eluted behind the original peak, and the masses at the peaks were 1139.0 and 1121.6. A mass of 1139.0 was almost identical to 1140.30 as the theoretical value with respect to a vasopressin-dichloroacetone-crosslinked form (vasopressin-DA) shown in the following formula B1, and thus a crosslinked product having an objective structure was determined to be included. On the other hand, a mass of 1121.6 was smaller than 1139.0 by 17.4, and the reason for this was presumed as follows: the vasopressin-DA shown in formula B1 underwent reduction reaction with TCEP and thus was formed into a reduced product (vasopressin-DA-R) of the vasopressin-dichloroacetone-crosslinked form, shown by the following formula B2, and furthermore underwent dehydration reaction and thus was formed into a reduced/dehydrated form (vasopressin-DA-RDH, theoretical value of mass: 1124.3) of the vasopressin-dichloroacetone-crosslinked form, shown by formula B3.

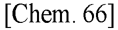

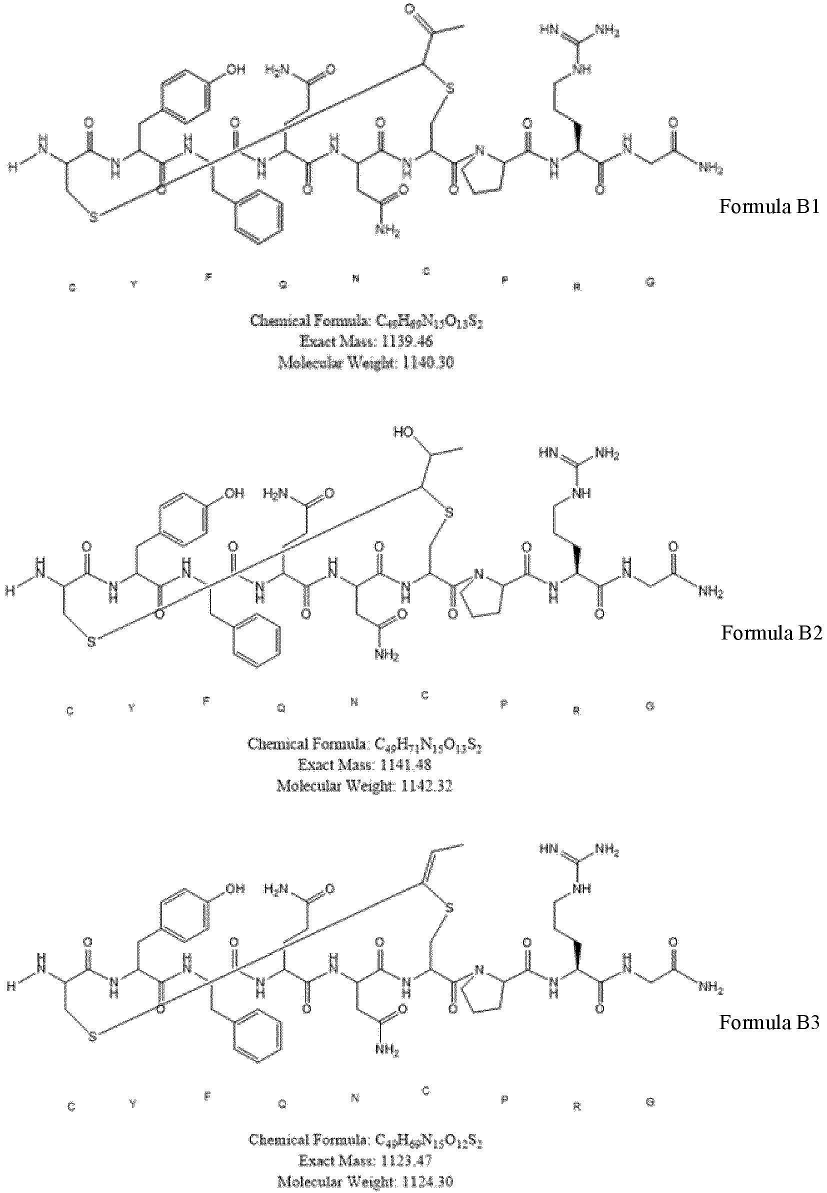

### (Example 13) Acquirement of resistance to protease by crosslinking of oxytocin

For evaluation of stability of a peptide by crosslinking reaction, resistivity of α-chymotrypsin (derived from bovine pancreas, manufactured by MP Biomedicals) to protease decomposition was evaluated with oxytocin crosslinked by 2,2-dichloroacetophenone. Crosslinking of oxytocin by 2,2-dichloroacetophenone was performed in the same manner with replacement of 1.1-dichloroacetone in Example 11 by 2,2-dichloroacetophenone, and an objective product was taken by reverse-phase HPLC. Specifically, 10 mg (10 µmol) of oxytocin acetate was dissolved in 3 mL of a 0.1 M HEPES hydrochloric acid buffer (pH 8.0), the solution was mixed with 2 mL of a 0.1 M HEPES hydrochloric acid buffer (pH 8.0) containing 57.4 mg (200 µmol) of TCEP-HCl, and the mixture was stirred for 1 hour. Next, 8.3 mg (44 µmol) of 2,2-dichloroacetophenone dissolved in 0.44 mL of acetonitrile was added and mixed, and the mixture was stirred at room temperature for 1 hour. The resulting sample was applied to an InertSustain C18 column (5 µm, 14 × 250 mm, manufactured by GL Sciences Inc.) (flow rate 5 mL/min) connected to LC-Forte (manufactured by YMC). The elution was performed at a linear gradient from 4% to 70% (containing 0.1% formic acid). An objective product was taken, and freeze-dried after removal of acetonitrile at a negative pressure.

A structure of a 2,2-dichloroacetophenone crosslinked form (oxytocin-DP) of oxytocin is shown by formula A2.

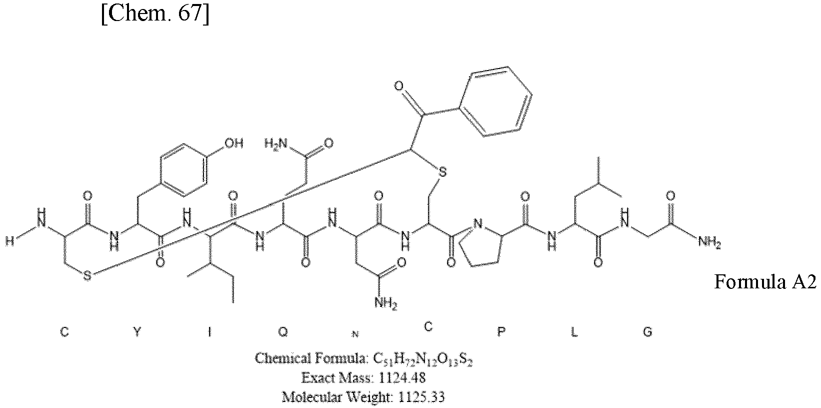

Evaluation was made with oxytocin-OX and an oxytocin SH reduced form (oxytocin-RD) shown by the following formula A3 used as comparison subjects. In other words, an oxytocin solution obtained by dissolution in a 0.1 M phosphate buffer (pH 7.0) at 0.5 mg/mL was adopted as oxytocin-OX, and a substance obtained after a lapse of 30 minutes from addition of an oxytocin solution obtained by dissolution in a 0.1 M phosphate buffer (pH 7.0) containing 0.5 mg/mL TCEP, at 0.5 mg/mL, was adopted as oxytocin-RD. To 200 µL of each of these solutions was 10 µL (1/10 on weight ratio to oxytocin) of 1 mg/mL α-chymotrypsin, and the resultant was subjected to incubation at 37°C and analysis by reverse-phase HPLC. In the case of blank, a phosphate buffer was added instead of α-chymotrypsin.

A substance obtained after a lapse of 30 minutes from dissolution of 0.5 mg/mL oxytocin-DP purified, in a 0.1 M phosphate buffer (pH 7.0) containing 0.5 mg/mL TCEP, was adopted as a sample, and similarly α-chymotrypsin was added and analysis by reverse-phase HPLC was made.

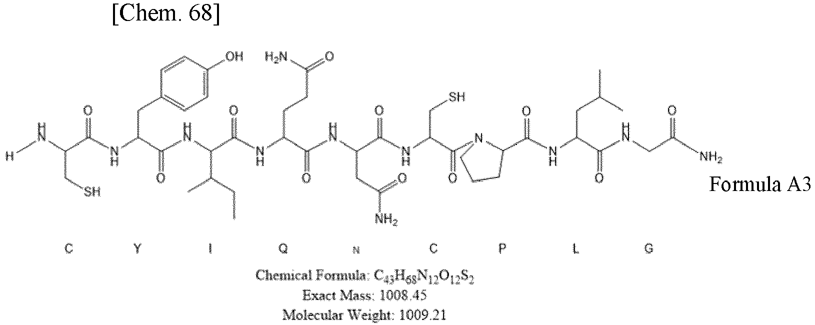

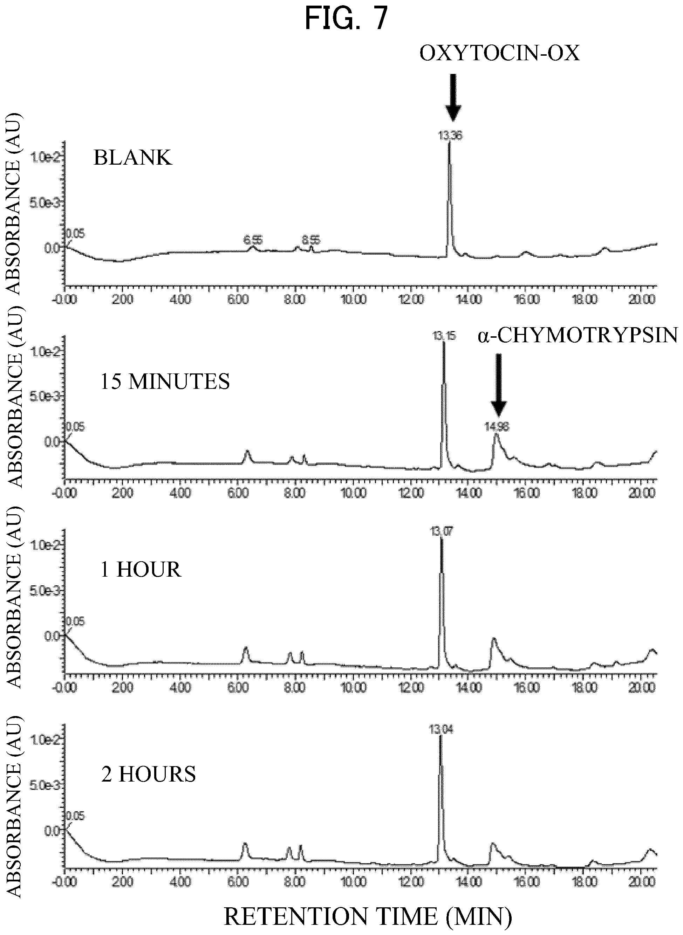

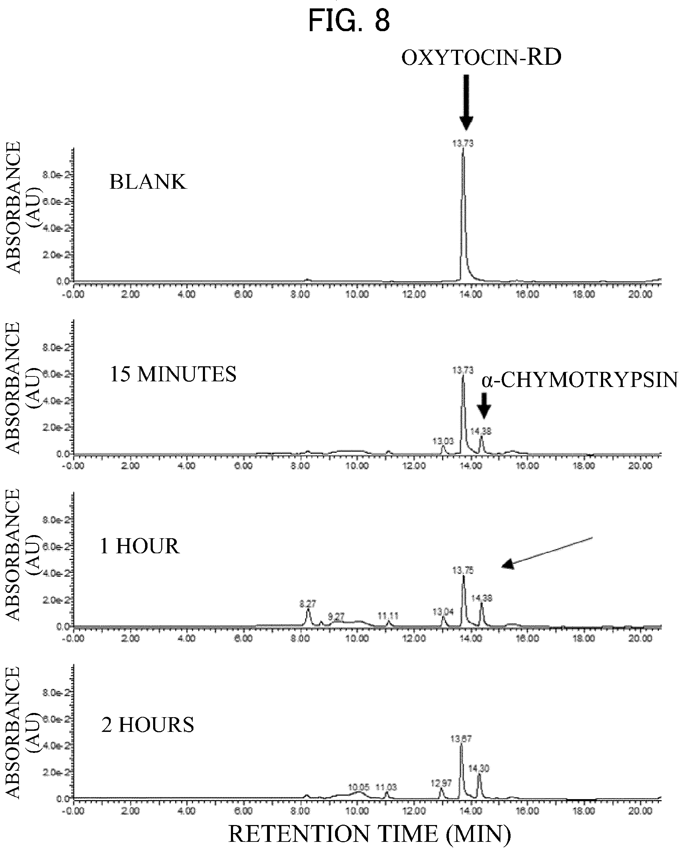

While no change of any peak was observed even after a lapse of 15 minutes to 2 hours in the case of oxytocin-OX, as illustrated in FIG. 7, any peak disappeared after only 10 minutes in the case of oxytocin-RD, as illustrated in FIG. 8. A broad peak eluted around 15 minutes was considered to be a peak assigned to α-chymotrypsin.

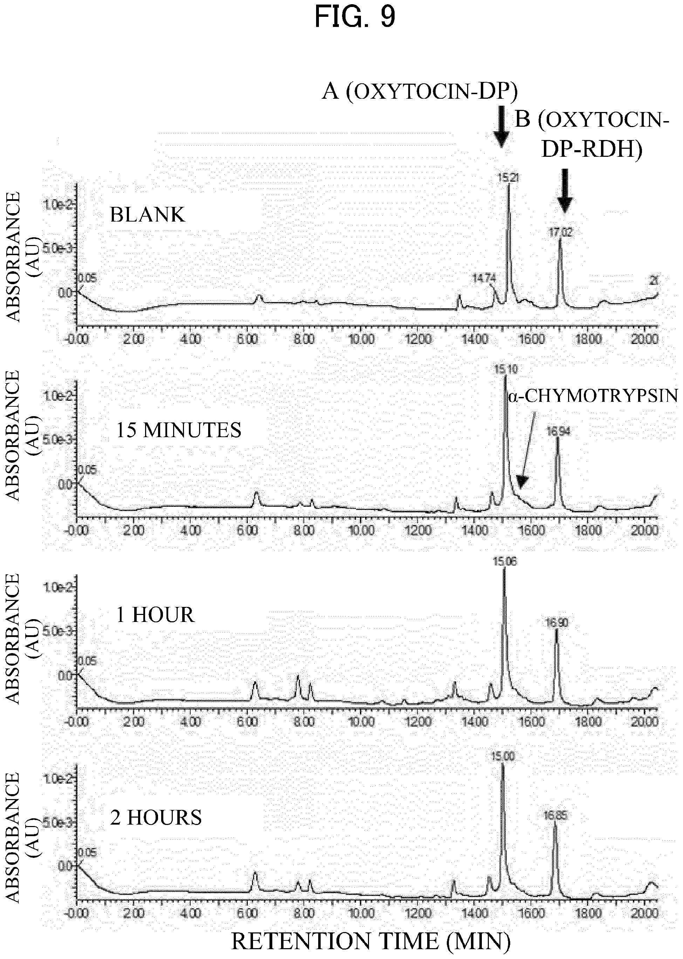

FIG. 9 illustrates an elution chromatogram of a sample prepared from oxytocin-DP, by reverse-phase HPLC. Here, two peaks, peaks A and B, appeared. The masses were analyzed by mass analysis, and thus the mass at peak A was 1126.7 and the mass at peak B was 1106.5. These were almost identical to 1125.34 as the theoretical value of the mass of an oxytocin-dichloroacetonephenone-crosslinked form (oxytocin-DP) shown by the following formula A4 and 1109.33 as the theoretical value of the mass of oxytocin-DP-RDH (the following formula A6) obtained by dehydration of a reduced product (oxytocin-DP-R, the following formula A5). It was thus found that the sample was a mixture of oxytocin-DP and oxytocin-DP-RDH. The reaction by addition of α-chymotrypsin in an amount corresponding to a weight ratio of 1/10, to this sample, was followed, and thus no decrease was observed in the two peaks even after addition of α-chymotrypsin, not like the case of oxytocin-RD, but like the case of oxytocin-OX.

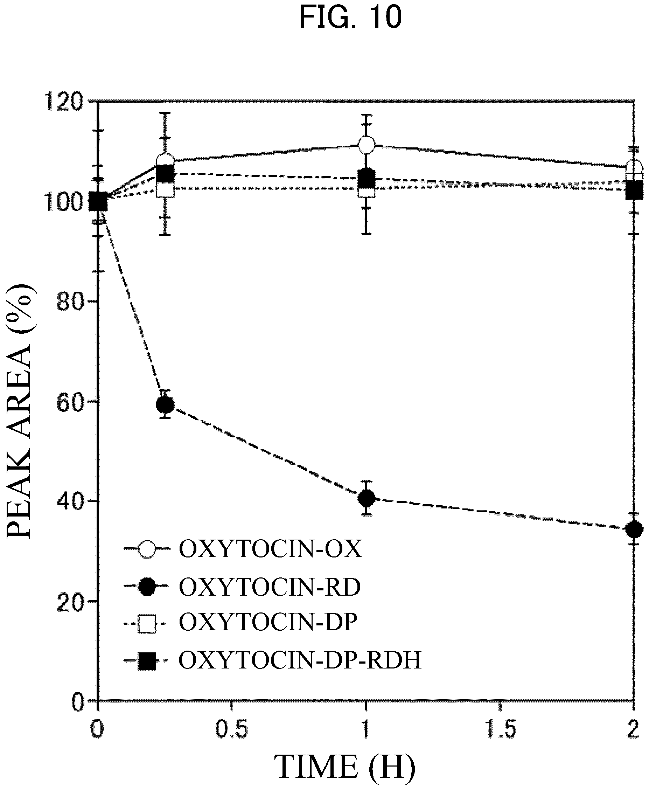

FIG. 10 illustrates the results of plotting of the relative value (%) to the peak area of blank against the time, in order to observe the amount of change with respect to each kind of these molecules after addition of α-chymotrypsin. While oxytocin-RD is considered to be extremely low in resistance to protease because of rapid disappearance of the peak immediately after addition of α-chymotrypsin, oxytocin-OX as an SS crosslinked product thereof is not observed to be decomposed even by addition of α-chymotrypsin and has high protease resistance, and thus crosslinking by an SS bond is found to largely contribute to resistance. On the other hand, the dichloroacetonephenone-crosslinked form (Oxytocin-DP and Oxytocin-DP-RDH) is also observed to have high resistance to α-chymotrypsin digestion in the presence of a reducing agent TCEP. This indicates that the present crosslinking method can largely contribute to stability of a peptide, in particular, resistance (stability) to protease.

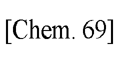

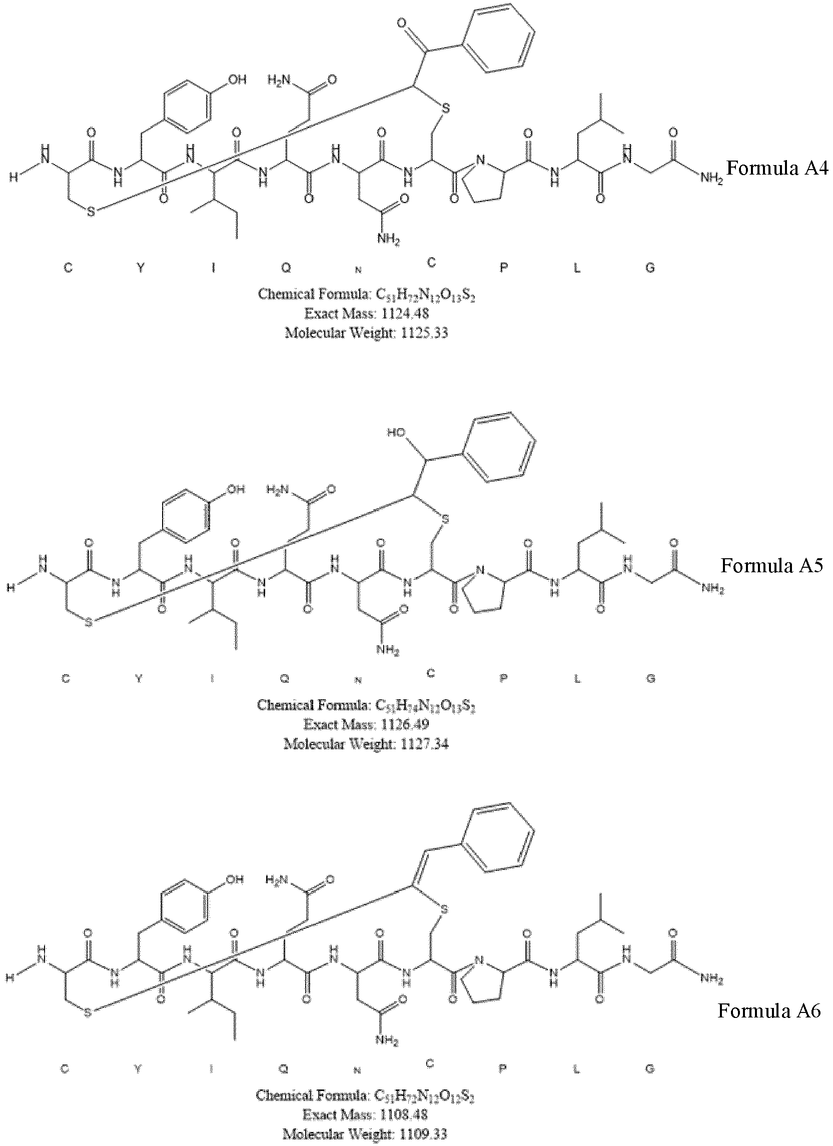

The foregoing describes some example embodiments for explanatory purposes. Although the foregoing discussion has presented specific embodiments, persons skilled in the art will recognize that changes may be made in form and detail without departing from the broader spirit and scope of the invention. Accordingly, the specification and drawings are to be regarded in an illustrative rather than a restrictive sense. This detailed description, therefore, is not to be taken in a limiting sense, and the scope of the invention is defined only by the included claims, along with the full range of equivalents where such claims are entitled.

This application claims the benefits of Japanese Patent Application No. 2020-186833, filed on November 9, 2020, and Japanese Patent Application No. 2021-82739 filed on May 14, 2021, the entire disclosures of which are incorporated by reference herein.

### Industrial Applicability

The present disclosure is useful for crosslinking a peptide and a protein.

Sequence List

## Claims

1. A crosslinking agent for a protein or a peptide, represented by the following formula (I): 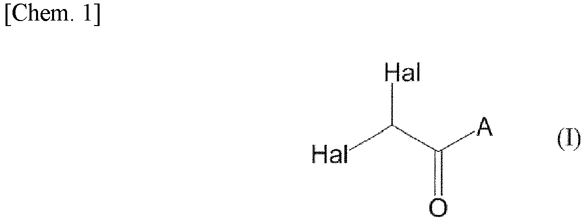 wherein A is a hydrogen atom, a C1 to 6 alkyl group optionally substituted with a phenyl group or a halogen atom, or a phenyl group.

2. A method for producing a crosslinked protein or peptide in which at least two thiol groups in a single or separated protein(s) or peptide(s) are bound as represented by the following formula: 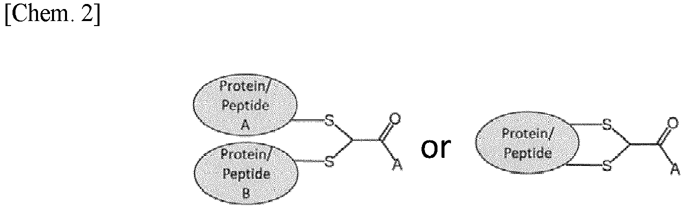
[wherein A is a hydrogen atom, a C1 to 6 alkyl group optionally substituted with a phenyl group or a halogen atom, a phenyl group, or a C1 to 6 alkyl group, Protein/Peptide represents a protein or a peptide, and Protein/Peptide A and Protein/Peptide B are optionally the same or different,]
comprising reacting the protein or the peptide with the crosslinking agent according to claim 1, to bind the two thiol groups via the following group: 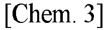 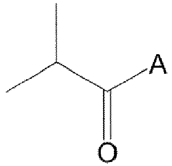
[wherein A is a hydrogen atom, a C1 to 6 alkyl group optionally substituted with a phenyl group or a halogen atom, a phenyl group, or a C1 to 6 alkyl group].

3. The method according to claim 2, wherein the protein or the peptide is a protein or a peptide wherein all or a part of the at least two thiol groups form a disulfide bond, and the method comprises reducing the disulfide bond to thereby generate two thiol groups.

4. A method for improving alkali resistance of a protein or a peptide, comprising binding thiol groups in the protein or the peptide to obtain a crosslinked protein or peptide by the method according to claim 2 or 3.

5. A method for producing a protein or a peptide bound to a reactive functional group which is represented by the following formula: 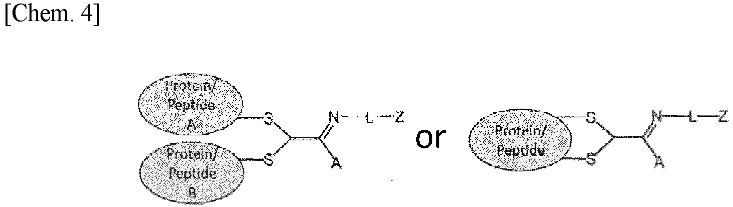 [wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and Z represents the reactive functional group;]
comprising:
obtaining the crosslinked protein or peptide by the method according to claim 2 or 3; and
reacting the crosslinked protein or peptide obtained, with NH₂-L-Z (L represents a linker and Z represents the reactive functional group), to thereby introduce a reactive functional group into a crosslinked portion of the crosslinked protein or peptide.

6. A method for producing a protein(s) or a peptide(s) bound to a reactive functional group which is represented by the following formula: 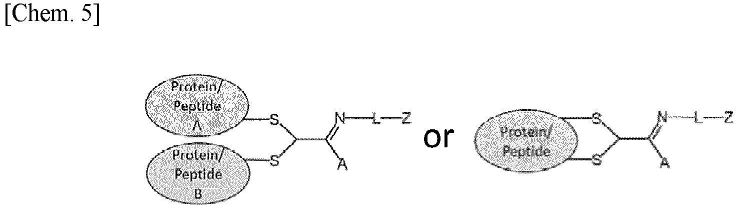
[wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and Z represents the reactive functional group;]
comprising:
reacting a crosslinked protein(s) or peptide(s) represented by the following formula: 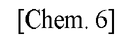 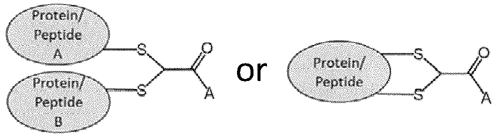
[wherein A is a hydrogen atom, a C1 to 6 alkyl group optionally substituted with a phenyl group or a halogen atom, a phenyl group, or a C1 to 6 alkyl group, Protein/Peptide represents a protein or a peptide, and Protein/Peptide A and Protein/Peptide B are optionally the same or different;]
and NH₂-L-Z (L represents a linker and Z represents the reactive functional group), to thereby introduce the reactive functional group to a crosslinked portion of the crosslinked protein(s) or peptide(s).

7. A method for producing a complex of a protein(s) or peptide(s) and a drug represented by the following formula: 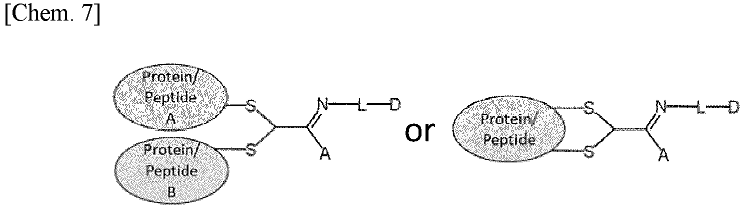 [wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and D represents the drug;]
comprising:
obtaining a protein(s) or a peptide(s) bound to a reactive functional group by the method according to claim 5 or 6; and
reacting the obtained protein(s) or peptide(s) bound to the reactive functional group and the drug having another functional group reactive with the reactive functional group, to thereby bind the drug to a crosslinked portion of the crosslinked protein(s) or peptide(s).

8. A method for producing a complex of a protein(s) or a peptide(s) and a drug represented by the following formula: 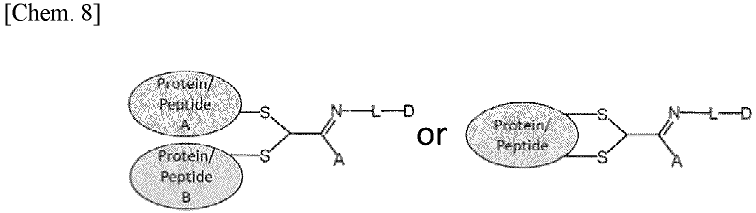
[wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and D represents the drug;]
comprising:
reacting a protein(s) or a peptide(s) bound to a reactive functional group which is represented by the following formula: 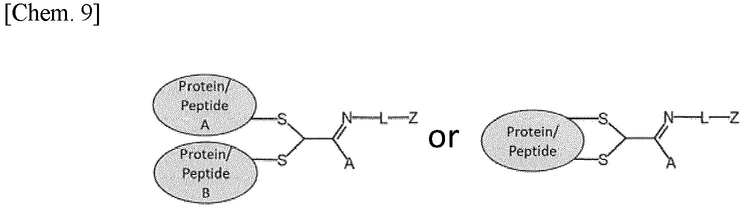
[wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and Z represents the reactive functional group;]
and the drug having another functional group reactive with the reactive functional group, to thereby bind the drug to a crosslinked portion of the crosslinked protein(s) or peptide(s).

9. The method according to any one of claims 5 to 8, wherein the linker contains a moiety cleavable by a proteolytic enzyme.

10. The method according to any one of claims 2 to 9, wherein the protein or the peptide is a peptide of 5 to 50 amino acids.

11. The method according to any one of claims 2 to 10, wherein the thiol groups for crosslinking are present in a single protein or peptide.

12. The method according to claim 11, wherein the protein or peptide is an Fc-binding peptide.

13. The method according to any one of claims 2 to 10, wherein the thiol groups for crosslinking are present in separated proteins or peptides.

14. A crosslinked protein(s) or peptide(s) represented by the following formula: 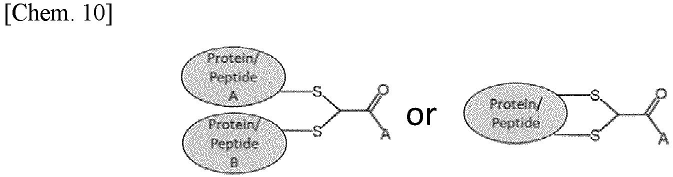 [wherein A is a hydrogen atom, a C1 to 6 alkyl group optionally substituted with a phenyl group or a halogen atom, a phenyl group, or a C1 to 6 alkyl group, Protein/Peptide represents a protein or a peptide, and Protein/Peptide A and Protein/Peptide B are optionally the same or different].

15. A protein(s) or a peptide(s) bound to a reactive functional group represented by the following formula: 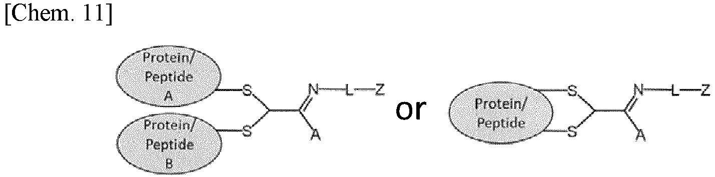 [wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and Z represents the reactive functional group.

16. A complex of a protein(s) or a peptide(s) and a drug represented by the following formula: 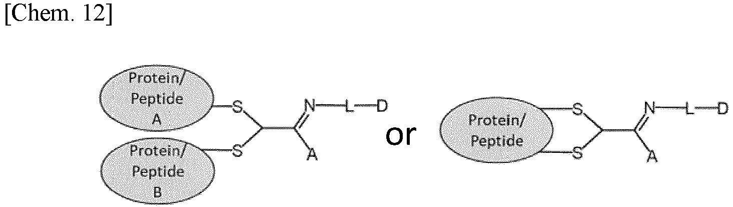 [wherein A is a hydrogen atom, a C1 to 6 alkyl group optionally substituted with a phenyl group or a halogen atom, a phenyl group, or a C1 to 6 alkyl group, Protein/Peptide represents a protein or a peptide, Protein/Peptide A and Protein/Peptide B are optionally the same or different, L represents a linker and D represents the drug].

17. The protein(s) or the peptide(s) according to claim 14, the protein(s) or peptide(s) according to claim 15, or the complex according to claim 16, wherein the linker contains a moiety cleavable by a proteolytic enzyme.

18. The protein, the peptide or the complex according to any one of claims 14 to 17, wherein the protein or the peptide is a peptide of 5 to 50 amino acids.

19. The protein, the peptide or the complex according to any one of claims 14 to 18, wherein the thiol groups for crosslinking are present in a single protein or peptide.

20. The protein, the peptide or the complex according to claim 19, wherein the protein or the peptide is an Fc-binding peptide.

21. The protein, the peptide or the complex according to claim 20, wherein an amino group in a lysine residue, a cysteine residue, an aspartic acid residue, a glutamic acid residue, 2-aminosuberic acid, diaminopropionic acid, an arginine residue or an amino acid at the 1-position in the Fc-binding peptide, is modified with DSG (disuccinimidyl glutarate), DSS (disuccinimidyl suberate), DMA (dimethyl adipimidate dihydrochloride), DMP (dimethyl pimelimidate dihydrochloride), DMS (dimethyl suberimidate dihydrochloride), DTBP (dimethyl 3,3'-dithiobispropionimidate dihydrochloride), or DSP (dithiobis(succinimidyl propionic acid)).

22. The protein, the peptide or the complex according to any one of claims 14 to 18, wherein the thiol groups for crosslinking are present in separated proteins or peptides.

23. A method for producing an IgG-Fc region-containing molecule bound to a crosslinked Fc-binding peptide(s), comprising contacting an IgG-Fc region-containing molecule and the protein, the peptide or the complex according to claim 20 or 21.

24. A method for producing an IgG-Fc region-containing molecule bound to a crosslinked Fc-binding peptide(s), comprising:
reacting an Fc-binding peptide bound to an IgG-Fc region-containing molecule, and the crosslinking agent according to claim 1, to bind two thiol groups in the Fc-binding peptide via the following group: 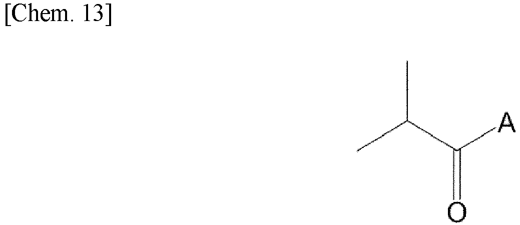
[wherein A is a hydrogen atom, a C1 to 6 alkyl group optionally substituted with a phenyl group or a halogen atom, a phenyl group, or a C1 to 6 alkyl group].

25. A method for producing an IgG-Fc region-containing molecule bound to an Fc-binding peptide having a reactive functional group Z,
wherein the Fc-binding peptide is represented by the following formula: 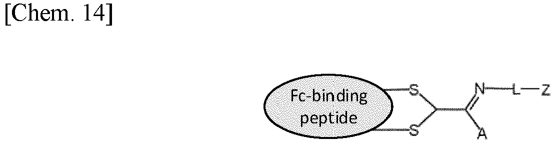
[wherein A is a hydrogen atom, a C1 to 6 alkyl group optionally substituted with a phenyl group or a halogen atom, a phenyl group, or a C1 to 6 alkyl group, L represents a linker and Z represents the reactive functional group;]
comprising:
reacting an IgG-Fc region-containing molecule bound to a crosslinked Fc-binding peptide represented by the following formula: 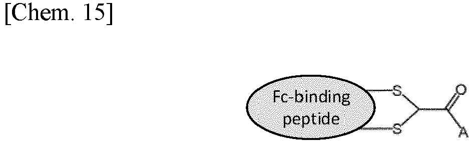
[wherein A is a hydrogen atom, a C1 to 6 alkyl group optionally substituted with a phenyl group or a halogen atom, a phenyl group, or a C1 to 6 alkyl group;] and NH₂-L-Z (L represents a linker and Z represents the reactive functional group), to thereby introduce the reactive functional group into a crosslinked portion in the crosslinked Fc-binding peptide.

26. A method for producing an IgG-Fc region-containing molecule bound to an Fc-binding peptide having a drug D,
wherein the Fc-binding peptide is represented by the following formula: 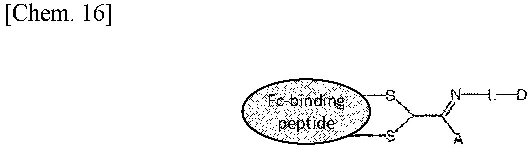 comprising:
reacting an IgG-Fc region-containing molecule bound to an Fc-binding peptide(s) having a reactive functional group Z,
wherein the Fc-binding peptide is represented by the following formula: 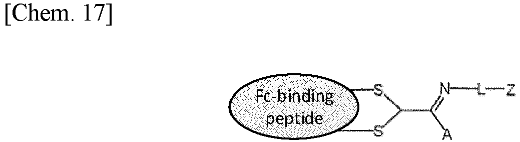
[wherein A is a hydrogen atom, a C1 to 6 alkyl group optionally substituted with a phenyl group or a halogen atom, a phenyl group, or a C1 to 6 alkyl group, L represents a linker and Z represents the reactive functional group;]
and the drug having another functional group reactive with the reactive functional group Z, to thereby bind the drug to a crosslinked portion of a crosslinked protein or peptide.

27. The method according to any one of claims 23 to 26, further comprising covalently binding the Fc-binding peptide and the IgG-Fc region-containing molecule.

28. An IgG-Fc region-containing molecule to which the protein, the peptide or the complex according to claim 20 or 21 is bound.

29. The molecule according to claim 28, wherein the Fc-binding peptide and the IgG-Fc region-containing molecule are covalently bound.

30. A carrier to which the peptide according to claim 20 or 21 is bound.

31. A method for purifying an IgG-Fc region-containing molecule, the method comprising:
contacting a liquid containing an IgG-Fc region-containing molecule, with the carrier according to claim 30;
removing a component not binding to the carrier, by washing; and
eluting a component bound to the carrier, to recover the IgG-Fc region-containing molecule.

32. A method for producing a crosslinked protein or peptide in which at least two thiol groups are bound as,
wherein the crosslinked protein or peptide is represented by the following formula: 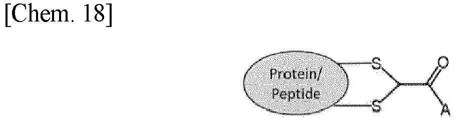
[wherein A is a hydrogen atom, a C1 to 6 alkyl group optionally substituted with a phenyl group or a halogen atom, a phenyl group, or a C1 to 6 alkyl group, and Protein/Peptide represents a protein or a peptide;]
comprising:
synthesizing the protein or peptide by an Fmoc method; and
using a compound represented by the following formula, instead of at least one cysteine residue, in the synthesis.

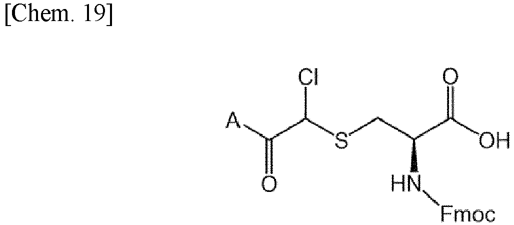

33. A method for producing a protein or a peptide bound to a reactive functional group represented by the following formula: 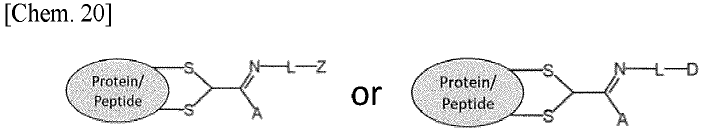 [wherein A is a hydrogen atom, a C1 to 6 alkyl group optionally substituted with a phenyl group or a halogen atom, a phenyl group, or a C1 to 6 alkyl group, Protein/Peptide represents a protein or a peptide, L represents a linker, Z represents the reactive functional group and D represents a drug;]
comprising:
synthesizing the protein or peptide by an Fmoc method; and
using a compound represented by the following formula, instead of at least one cysteine residue, in the synthesis.
